# EUROPÄISCHE PATENTANMELDUNG

(11) **EP 1 905 765 A1**
(43) Veröffentlichungstag der Anmeldung: **02.04.2008**
(21) Anmeldenummer: 07121390.4
(22) Anmeldetag: 16.06.2004
(51) Int. Cl.: C07D 239/42, C07D 401/12, C07D 409/12, A61K 31/505, A61K 31/506, A61P 25/28

(54) **6-Aylamino-5-Cyano-4-Pyrimidinone als PDE9A-Inhibitoren**

(30) Priorität: 25.06.2003 DE 10328479
(62) Teilanmeldung aus: 04739944.9
(71) Anmelder: Boehringer Ingelheim International GmbH, 55218 Ingelheim am Rhein (DE)
(72) Erfinder: Hendrix, Martin, 51519 Odenthal (DE); Bärfacker, Lars, 46047 Oberhausen (DE); Beyreuther, Bettina, 40219 Düsseldorf (DE); Ebert, Ulrich, 68163 Mannheim (DE); Erb, Christina, 65830 Kriftel (DE); Hafner, Frank-Thorsten, 42115 Wuppertal (DE); Heckroth, Heike, 51519 Odenthal (DE); Liu, Yan-Hong, 60316 Frankfurt (DE); Karthaus, Dagmar, 42697 Solingen (DE); Tersteegen, Adrian, 42115 Wuppertal (DE)
(74) Vertreter: Fuchs, Georg Ludwig

(57) **Zusammenfassung**

Die Erfindung betrifft neue 6-Arylamino-5-cyano-4-pyrimidinone der Formel (I), Verfahren zu ihrer Herstellung, und ihre Verwendung zur Herstellung von Arzneimitteln zur Verbesserung von Wahrnehmung, Konzentrationsleistung, Lern- und/oder Gedächtnisleistung.

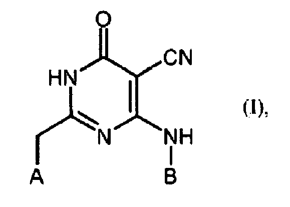

## Beschreibung

Die Erfindung betrifft neue 6-Arylamino-5-cyano-4-pyrimidinone, Verfahren zu ihrer Herstellung, und ihre Verwendung zur Herstellung von Arzneimitteln zur Verbesserung von Wahrnehmung, Konzentrationsleistung, Lern- und/oder Gedächtnisleistung.

Inhibition von Phosphordiesterasen moduliert die Spiegel der zyklischen Nukleotide 5'-3' zyklisches Adenosinmonophosphat (cAMP) bzw. 5'-3' zyklisches Guanosinmonophosphat (cGMP). Diese zyklischen Nukleotide (cAMP und cGMP) sind wichtige second messenger und spielen daher eine zentrale Rolle in den zellulären Signaltransduktionskaskaden. Beide aktivieren unter anderem, aber nicht ausschließlich, jeweils wieder Proteinkinasen. Die von cAMP aktivierte Proteinkinase wird Proteinkinase A (PKA) genannt, die von cGMP aktivierte Proteinkinase wird Proteinkinase G (PKG) genannt. Aktivierte PKA bzw. PKG können wiederum eine Reihe zellulärer Effektorproteine phosphorylieren (z.B. Ionenkanäle, G-Protein gekoppelte Rezeptoren, Strukturproteine). Auf diese Weise können die second messengers cAMP und cGMP die unterschiedlichsten physiologischen Vorgänge in den verschiedensten Organen kontrollieren. Die zyklischen Nukleotide können aber auch direkt auf Effektormoleküle wirken. So ist z.B. bekannt, dass cGMP direkt auf lonenkanäle wirken kann und hiermit die zelluläre Ionenkonzentration beeinflussen kann (Übersicht in: Wei et al., Prog. Neurobiol., 1998, 56: 37 - 64). Ein Kontrollmechanismus, um die Aktivität von cAMP und cGMP und damit diese physiologischen Vorgänge wiederum zu steuern, sind die Phosphodiesterasen (PDE). PDEs hydrolysieren die zyklischen Monophosphate zu den inaktiven Monophosphaten AMP und GMP. Es sind mittlerweile mindestens 21 PDE Gene beschrieben (Exp. Opin. Investig. Drugs 2000, 9, 1354-3784). Diese 21 PDE Gene lassen sich aufgrund ihrer Sequenzhomologie in 11 PDE Familien einteilen (Nomenklatur Vorschlag siehe http://depts.washington.edu/pde/Nomenclature.html.). Einzelne PDE Gene innerhalb einer Familie werden durch Buchstaben unterschieden (z.B. PDE1A und PDE1B). Falls noch unterschiedliche Splice Varianten innerhalb eines Genes vorkommen, wird dies dann durch eine zusätzliche Nummerierung nach dem Buchstaben angegeben (z.B. PDE1A1).

Die Humane PDE9A wurde 1998 kloniert und sequenziert. Die Aminosäurenidentität zu anderen PDEs liegt bei maximal 34 % (PDE8A) und minimal 28 % (PDE5A). Mit einer Michaelis-Menten-Konstante (Km-Wert) von 170 nM ist PDE9A hochaffin für cGMP. Darüber hinaus ist PDE9A selektiv für cGMP (Km-Wert für cAMP = 230 µM). PDE9A weist keine cGMP Bindungsdomäne auf, die auf eine allosterische Enzymregulation durch cGMP schließen ließe. In einer Western Blot Analyse wurde gezeigt, dass die PDE9A im Mensch unter anderem in Hoden, Gehirn, Dünndarm, Skelettmuskulatur, Herz, Lunge, Thymus und Milz exprimiert wird. Die höchste Expression wurde in Gehirn, Dünndarm, Herz und Milz gefunden (Fisher et al., J. Biol. Chem., 1998, 273 (25): 15559 - 15564). Das Gen für die humane PDE9A liegt auf Chromosom 21q22.3 und enthält 21 Exons. Bislang wurden 4 alternative Spleißvarianten der PDE9A identifiziert (Guipponi et al., Hum. Genet., 1998, 103: 386 - 392). Klassische PDE Inhibitoren hemmen die humane PDE9A nicht. So zeigen IBMX, Dipyridamole, SKF94120, Rolipram und Vinpocetin in Konzentrationen bis 100 µM keine Inhibition am isolierten Enzym. Für Zaprinast wurde ein IC₅₀-Wert von 35 µM nachgewiesen (Fisher et al., J. Biol. Chem., 1998, 273 (25): 15559 - 15564).

Die Maus PDE9A wurde 1998 von Soderling et al. (J. Biol. Chem., 1998, 273 (19): 15553 - 15558) kloniert und sequenziert. Diese ist wie die humane Form hochaffin für cGMP mit einem Km von 70 nM. In der Maus wurde eine besonders hohe Expression in der Niere, Gehirn, Lunge und Herz gefunden. Auch die Maus PDE9A wird von IBMX in Konzentrationen unter 200 µM nicht gehemmt; der IC₅₀-Wert für Zaprinast liegt bei 29 µM (Soderling et al., J. Biol. Chem., 1998, 273 (19): 15553 - 15558). Im Rattengehirn wurde gezeigt, dass PDE9A in einigen Hirnregionen stark exprimiert wird. Dazu zählen der Bulbus olfactorius, Hippocampus, Cortex, Basalganglien und basales Vorderhirn (Andreeva et al., J. Neurosci., 2001, 21 (22): 9068 - 9076). Insbesondere Hippocampus, Cortex und basales Vorderhirn spielen eine wichtige Rolle an Lern- und Gedächtnisvorgängen.

Wie oben bereits erwähnt, zeichnet sich PDE9A durch eine besonders hohe Affinität für cGMP aus. Deshalb ist PDE9A im Gegensatz zu PDE2A (Km = 10 µM; Martins et al., J. Biol. Chem., 1982, 257: 1973 - 1979), PDE5A (Km = 4 µM; Francis et al., J. Biol. Chem., 1980, 255: 620 - 626), PDE6A (Km = 17 µM; Gillespie and Beavo, J. Biol. Chem., 1988, 263 (17): 8133 - 8141) und PDE11A (Km = 0,52 µM; Fawcett et al., Proc. Nat. Acad. Sci., 2000, 97 (7): 3702 - 3707) schon bei niedrigen physiologischen Konzentrationen aktiv. Im Gegensatz zu PDE2A (Murashima et al., Biochemistry, 1990, 29: 5285 - 5292) wird die katalytische Aktvität von PDE9A nicht durch cGMP gesteigert, da es keine GAF Domäne (cGMP Bindedomäne, über die die PDE Aktivität allosterisch gesteigert wird) aufweist (Beavo et al., Current Opinion in Cell Biology, 2000, 12: 174 -179). PDE9A Inhibitoren können deshalb zu einer Erhöhung der basalen cGMP Konzentration führen.

Die US 5,256,668 offenbart Aminopyrimidin-Derivate, die sich als antivirale Mittel auszeichnen und für die Behandlung des Respiratory Syncytial Virus eingesetzt werden können.

In WO 99/41253 werden Pyrimidin-Derivate mit antiviraler Wirkung beschrieben, die besonders zur Behandlung von humanen Cytomegalovirusinfektionen eingesetzt werden können.

Die EP 130735 offenbart Aminopyrimidin-Derivate, die sich als herzstärkende Mittel auszeichnen.

Die vorliegende Erfindung betrifft Verbindungen der Formel 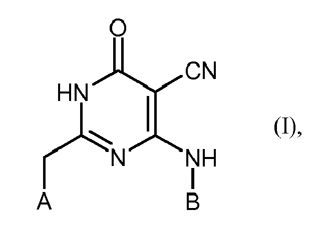 in welcher
- A: C₁-C₈-Alkyl, C₃-C₈-Cycloalkyl, Tetrahydrofuryl oder Tetrahydropyranyl, welche gegebenenfalls mit bis zu 3 Resten unabhängig voneinander ausgewählt aus der Gruppe C₁-C₆-Alkyl, C₁-C₆-Alkoxy, Hydroxycarbonyl, Cyano, Trifluormethyl, Trifluormethoxy, Amino, Hydroxy, C₁-C₆-Alkylamino, Halogen, C₁-C₆-Alkylaminocarbonyl, C₁-C₆-Alkoxycarbonyl, C₁-C₆-Alkylcarbonyl, C₁-C₆-Alkylsulfonyl und C₁-C₆-Alkylthio substituiert sind,
wobei C₁-C₆-Alkyl, C₁-C₆-Alkoxy, C₁-C₆-Alkylamino, C₁-C₆-Alkylaminocarbonyl, C₁-C₆-Alkoxycarbonyl, C₁-C₆-Alkylcarbonyl, C₁-C₆-Alkylsulfonyl und C₁-C₆-Alkylthio gegebenenfalls mit einem oder mehreren Resten ausgewählt aus der Gruppe Hydroxy, Cyano, Halogen, Hydroxycarbonyl und einer Gruppe der Formel -NR³R⁴,
wobei
R³ und R⁴ unabhängig voneinander Wasserstoff oder C₁-C₆-Alkyl,
oder
R³ und R⁴ zusammen mit dem Stickstoffatom, an das sie gebunden sind, 5- bis 8-gliedriges Heterocyclyl bedeuten,
substituiert sind,
- B: Phenyl oder Heteroaryl, die gegebenenfalls mit bis zu 3 Resten jeweils unabhängig voneinander ausgewählt aus der Gruppe C₁-C₆-Alkyl, C₁-C₆-Alkoxy, Hydroxycarbonyl, Cyano, Trifluormethyl, Trifluormethoxy, Amino, Nitro, Hydroxy, C₁-C₆-Alkylamino, Halogen, C₁-C₆-Alkylaminocarbonyl, C₁-C₆-Alkoxycarbonyl, C₁-C₆-Alkylcarbonyl, C₁-C₆-Alkylsulfonyl und C₁-C₆-Alkylthio substituiert sind,
wobei C₁-C₆-Alkyl, C₁-C₆-Alkoxy, C₁-C₆-Alkylamino, C₁-C₆-Alkylaminocarbonyl, C₁-C₆-Alkoxycarbonyl, C₁-C₆-Alkylcarbonyl, C₁-C₆-Alkylsulfonyl und C₁-C₆-Alkylthio gegebenenfalls mit einem Rest ausgewählt aus der Gruppe Hydroxy, Cyano, Halogen, Hydroxycarbonyl und einer Gruppe der Formel -NR³R⁴,
wobei
R³ und R⁴ die oben angegebenen Bedeutungen aufweisen,
substituiert sind,
bedeuten, sowie deren Salze, Solvate und/oder Solvate der Salze.

Die erfindungsgemäßen Verbindungen können in Abhängigkeit von ihrer Struktur in stereoisomeren Formen (Enantiomere, Diastereomere) und tautomeren Formen existieren. Die Erfindung betrifft deshalb die Enantiomeren oder Diastereomeren und ihre jeweiligen Mischungen. Aus solchen Mischungen von Enantiomeren und/oder Diastereomeren lassen sich die stereoisomer einheitlichen Bestandteile in bekannter Weise isolieren.

Als Salze sind im Rahmen der Erfindung physiologisch unbedenkliche Salze der erfindungsgemäßen Verbindungen bevorzugt.

Physiologisch unbedenkliche Salze der Verbindungen (I) umfassen Säureadditionssalze von Mineralsäuren, Carbonsäuren und Sulfonsäuren, z.B. Salze der Chlorwasserstoffsäure, Bromwasserstoffsäure, Schwefelsäure, Phosphorsäure, Methansulfonsäure, Ethansulfonsäure, Toluolsulfonsäure, Benzolsulfonsäure, Naphthalindisulfonsäure, Essigsäure, Propionsäure, Milchsäure, Weinsäure, Äpfelsäure, Zitronensäure, Fumarsäure, Maleinsäure und Benzoesäure.

Physiologisch unbedenkliche Salze der Verbindungen (I) umfassen auch Salze üblicher Basen, wie beispielhaft und vorzugsweise Alkalimetallsalze (z.B. Natrium- und Kaliumsalze), Erdalkalisalze (z.B. Calcium- und Magnesiumsalze) und Ammoniumsalze, abgeleitet von Ammoniak oder organischen Aminen mit 1 bis 16 C-Atomen, wie beispielhaft und vorzugsweise Ethylamin, Diethylamin, Triethylamin, Ethyldiisopropylamin, Monoethanolamin, Diethanolamin, Triethanolamin, Dicyclohexylamin, Dimethylaminoethanol, Prokain, Dibenzylamin, N-Methylmorpholin, Dehydroabietylamin, Arginin, Lysin, Ethylendiamin und Methylpiperidin.

Als Solvate werden im Rahmen der Erfindung solche Formen der Verbindungen bezeichnet, welche in festem oder flüssigem Zustand durch Koordination mit Lösungsmittelmolekülen einen Komplex bilden. Hydrate sind eine spezielle Form der Solvate, bei denen die Koordination mit Wasser erfolgt.

Im Rahmen der vorliegenden Erfindung haben die Substituenten, soweit nicht anders spezifiziert, die folgende Bedeutung:
C₁-C₈-Alkyl, C₁-C₆-Alkyl, C₁-C₅-Alkyl und C₁-C₄-Alkyl stehen für einen geradkettigen oder verzweigten Alkylrest mit 1 bis 8, bevorzugt 1 bis 6, besonders bevorzugt 1 bis 5 und 1 bis 4 Kohlenstoffatomen. Bevorzugte Beispiele umfassen Methyl, Ethyl, n-Propyl, Isopropyl, 2-Butyl, 2-Pentyl und 3-Pentyl.
C₁-C₆-Alkoxy steht für einen geradkettigen oder verzweigten Alkoxyrest mit 1 bis 6, bevorzugt 1 bis 4, besonders bevorzugt mit 1 bis 3 Kohlenstoffatomen. Bevorzugte Beispiele umfassen Methoxy, Ethoxy, n-Propoxy, Isopropoxy, tert.-Butoxy, n-Pentoxy und n-Hexoxy.
C₁-C₆-Alkoxycarbonyl steht für einen geradkettigen oder verzweigten Alkoxycarbonylrest mit 1 bis 6, bevorzugt 1 bis 4 und besonders bevorzugt 1 bis 3 Kohlenstoffatomen. Bevorzugte Beispiele umfassen Methoxycarbonyl, Ethoxycarbonyl, n-Propoxycarbonyl, Isopropoxycarbonyl und tert.Butoxycarbonyl.
C₁-C₆-Alkylamino steht für einen geradkettigen oder verzweigten Mono- oder Dialkylaminorest mit 1 bis 6, bevorzugt 1 bis 4 und besonders bevorzugt mit 1 bis 3 Kohlenstoffatomen. Bevorzugte Beispiele umfassen Methylamino, Ethylamino, n-Propylamino, Isopropylamino, tert.-Butylamino, n-Pentylamino und n-Hexylamino, Dimethylamino, Diethylamino, Di-n-propylamino, Diisopropylamino, Di-tert.-butyl-amino, Di-n-pentylamino, Di-n-hexylamino, Ethyhnethylamino, Isopropylmethylamino, n-Butylethylamino und n-Hexyl-i-pentylamino.
C₁-C₆-Alkylaminocarbonyl steht für einen über eine Carbonyl-Gruppe verknüpften Mono- oder Dialkylaminorest, wobei die Alkylreste gleich oder verschieden sein können, geradkettig oder verzweigt sind und jeweils 1 bis 6, bevorzugt 1 bis 4, und besonders bevorzugt 1 bis 3 Kohlenstoffatome enthalten. Bevorzugte Beispiele umfassen Methylaminocarbonyl, Ethylaminocarbonyl, n-Propylaminocarbonyl, Isopropylaminocarbonyl, tert.Butylaminocarbonyl, n-Pentylaminocarbonyl, n-Hexylaminocarbonyl, Dimethylaminocarbonyl, Diethylaminocarbonyl, Di-n-propylaminocarbonyl, Diisopropylaminocarbonyl, Di-t-butylaminocarbonyl, Di-n-pentylaminocarbonyl, Di-n-hexylaminocarbonyl, Ethyhnethylaminocarbonyl, Isopropylmethylaminocarbonyl, n-Butylethylaminocarbonyl und n-Hexyl-i-pentylaminocarbonyl. Weiterhin können im Falle eines Dialkylaminorestes die beiden Alkylreste zusammen mit dem Stickstoffatom, an das sie gebunden sind, einen 5- bis 8-gliedriges Heterocyclyl bilden.
C₁-C₆-Alkylcarbonyl steht für einen geradkettigen oder verzweigten Alkylcarbonylrest mit 1 bis 6 und bevorzugt 1 bis 4 Kohlenstoffatomen. Beispielsweise seien genannt: Acetyl, Ethylcarbonyl, Propylcarbonyl, Isopropylcarbonyl, Butylcarbonyl, Isobutylcarbonyl, Pentylcarbonyl und Hexylcarbonyl. Besonders bevorzugt sind Acetyl und Ethylcarbonyl.
C₁₋C₆-Alkylsulfonyl steht für einen geradkettigen oder verzweigten Alkylsulfonylrest mit 1 bis 6, bevorzugt 1 bis 4 und besonders bevorzugt mit 1 bis 3 Kohlenstoffatomen. Bevorzugte Beispiele umfassen Methylsulfonyl, Ethylsulfonyl, n-Propylsulfonyl, Isopropylsulfonyl, tert.Butylsulfonyl, n-Pentylsulfonyl und n-Hexylsulfonyl.
C₁-C₆-Alkylthio steht für einen geradkettigen oder verzweigten Alkylthiorest mit 1 bis 6, bevorzugt 1 bis 4 und besonders bevorzugt mit 1 bis 3 Kohlenstoffatomen. Bevorzugte Beispiele umfassen Methylthio, Ethylthio, n-Propylthio, Isopropylthio, tert.Butylthio, n-Pentylthio und n-Hexylthio.
Halogen steht für Fluor, Chlor, Brom und Iod. Bevorzugt sind Fluor, Chlor, Brom, besonders bevorzugt Fluor und Chlor.
Heteroaryl steht für einen aromatischen, monocyclischen Rest mit 5 bis 6 Ringatomen und bis zu 3 Heteroatomen aus der Reihe S, O und/oder N. Bevorzugt sind 5- bis 6-gliedrige Heteroaryle mit bis zu 2 Heteroatomen. Der Heteroarylrest kann über ein Kohlenstoff- oder Stickstoffatom gebunden sein. Bevorzugte Beispiele umfassen Thienyl, Furyl, Pyrrolyl, Thiazolyl, Oxazolyl, Imidazolyl, Tetrazolyl, Pyridyl, Pyrimidinyl und Pyridazinyl.
3- bis 8-gliedriges Cycloalkyl steht für gesättigte und teilweise ungesättigte nicht-aromatische Cycloalkylreste mit 3 bis 8, bevorzugt 3 bis 6 und besonders bevorzugt 5 bis 6 Kohlenstoffatomen im Cyclus. Bevorzugte Beispiele umfassen Cyclopropyl, Cyclobutyl, Cyclopentyl, Cyclopentenyl, Cyclohexyl und Cyclohexenyl.
5- bis 8-gliedriges Heterocyclyl steht für einen mono- oder polycyclischen, heterocyclischen Rest mit 5 bis 8 Ringatomen und bis zu 3, vorzugsweise 2 Heteroatomen bzw. Heterogruppen aus der Reihe N, O, S, SO, SO₂. Mono- oder bicyclisches Heterocyclyl ist bevorzugt. Besonders bevorzugt ist monocyclisches Heterocyclyl. Als Heteroatome sind N und O bevorzugt. Die Heterocyclyl-Reste können gesättigt oder teilweise ungesättigt sein. Gesättigte Heterocyclyl-Reste sind bevorzugt. Besonders bevorzugt sind 5- bis 7-gliedrige Heterocyclylreste. Bevorzugte Beispiele umfassen Oxetan-3-yl, Pyrrolidin-2-yl, Pyrrolidin-3-yl, Pyrrolinyl, Tetrahydrofuranyl, Tetrahydrothienyl, Pyranyl, Piperidinyl, Thiopyranyl, Morpholinyl, Perhydroazepinyl.

Wenn Reste in den erfindungsgemäßen Verbindungen gegebenenfalls substituiert sind, ist, soweit nicht anders spezifiziert, eine Substitution mit bis zu drei gleichen oder verschiedenen Substituenten bevorzugt.

Eine weitere Ausführungsform der Erfindung betrifft Verbindungen der Formel (I),
in welcher
- A: C₁-C₅-Alkyl oder C₃-C₆-Cycloalkyl, welche gegebenenfalls mit bis zu 3 Resten unabhängig voneinander ausgewählt aus der Gruppe C₁-C₄-Alkyl, C₁-C₄-Alkoxy, Hydroxycarbonyl, Cyano, Amino, Hydroxy, C₁-C₄-Alkylamino, Fluor, Chlor, Brom, C₁-C₄-Alkoxycarbonyl, C₁-C₆-Alkylcarbonyl, C₁-C₄-Alkylsulfonyl und C₁-C₄-Alkylthio substituiert sind,
wobei C₁-C₄-Alkyl und C₁-C₄-Alkoxy gegebenenfalls mit einem Rest ausgewählt aus der Gruppe Hydroxy, Cyano, Fluor, Chlor, Brom, Hydroxycarbonyl und einer Gruppe der Formel -NR³R⁴,
wobei
R³ und R⁴ unabhängig voneinander Wasserstoff oder C₁-C₄-Alkyl,
oder
R³ und R⁴ zusammen mit dem Stickstoffatom, an das sie gebunden sind, 5- bis 6-gliedriges Heterocyclyl bedeuten,
substituiert sind,
- B: Phenyl, Thienyl oder Pyridyl, die gegebenenfalls mit bis zu 3 Resten jeweils unabhängig voneinander ausgewählt aus der Gruppe C₁-C₄-Alkyl, C₁-C₄-Alkoxy, Hydroxycarbonyl, Cyano, Trifluormethyl, Trifluormethoxy, Amino, Hydroxy, C₁-C₄-Alkylamino, Fluor, Chlor, Brom, C₁-C₄-Alkylaminocarbonyl, C₁-C₄-Alkoxycarbonyl, C₁-C₄-Alkylcarbonyl, C₁-C₄-Alkylsulfonyl und C₁-C₄-Alkylthio substituiert sind,
wobei C₁-C₄-Alkyl und C₁-C₄-Alkoxy gegebenenfalls mit einem Rest ausgewählt aus der Gruppe Hydroxy, Cyano, Fluor, Chlor, Brom, Hydroxycarbonyl und einer Gruppe der Formel -NR³R⁴,
wobei
R³ und R⁴ die oben angegebenen Bedeutungen aufweisen,
substituiert sind,
bedeuten, sowie deren Salze, Solvate und/oder Solvate der Salze.

Eine weitere Ausführungsform der Erfindung betrifft Verbindungen der Formel (I),
in welcher
- A: die oben angegebenen Bedeutungen aufweist, und
- B: Phenyl oder Pyridyl, die gegebenenfalls mit bis zu 3 Resten jeweils unabhängig voneinander ausgewählt aus der Gruppe Methyl, Ethyl, 2-Propyl, Trifluormethyl, Methoxy, Ethoxy, Fluor und Chlor,
wobei einer der Reste am Phenyl oder Pyridyl in ortho-Position relativ zur Anknüpfungsstelle der Aminofunktion lokalisiert ist,
substituiert sind,
bedeutet, sowie deren Salze, Solvate und/oder Solvate der Salze.

Eine weitere Ausführungsform der Erfindung betrifft Verbindungen der Formel (I),
in welcher
- A: C₃-C₆-Cycloalkyl bedeutet und
- B: die oben angegebenen Bedeutungen aufweist,
bedeuten, sowie deren Salze, Solvate und/oder Solvate der Salze.

Eine weitere Ausführungsform der Erfindung betrifft Verbindungen der Formel (I),
in welcher
- A: 2-Methylpropyl, 2-Butyl, 2-Pentyl oder 3-Pentyl bedeutet und
- B: die oben angegebenen Bedeutungen aufweist,
sowie deren Salze, Solvate und/oder Solvate der Salze.

Eine weitere Ausführungsform der Erfindung betrifft Verbindungen der Formel (I),
in welcher
- A: C₃-C₅-Alkyl oder C₅-C₆-Cycloalkyl,
- B: Phenyl, Thienyl oder Pyridyl, die gegebenenfalls mit bis zu 3 Resten jeweils unabhängig voneinander ausgewählt aus der Gruppe C₁-C₃-Alkyl, Trifluormethyl, Hydroxy, Methoxy, Ethoxy, Cyano, Dimethylamino, Diethylamino, Methoxycarbonyl, Ethoxycarbonyl, Methylcarbonyl, Ethylcarbonyl, Fluor und Chlor substituiert sind,
bedeuten, sowie deren Salze, Solvate und/oder Solvate der Salze.

Außerdem wurde ein Verfahren zur Herstellung der erfindungsgemäßen Verbindungen der Formel (I) gefunden, dadurch gekennzeichnet, dass man eine Verbindung der Formel 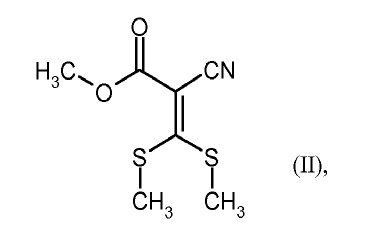 zunächst mit einer Verbindung der Formel

H₂N-B (III)

,
in welcher
- B: die oben angegebenen Bedeutungen aufweist,
bei erhöhter Temperatur in einem inerten Lösemittel oder auch in Abwesenheit eines Lösemittels in eine Verbindung der Formel 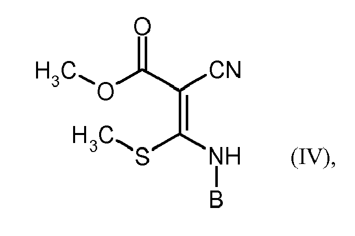 in welcher
- B: die oben angegebenen Bedeutungen aufweist,
überführt und diese dann in einem inerten Lösemittel in Gegenwart einer Base mit einer Verbindung der Formel 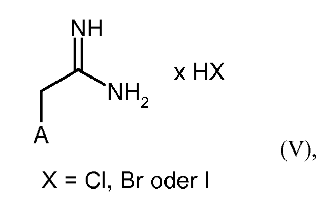 in welcher
- A: die oben angegebenen Bedeutungen aufweist,
umsetzt,
und die resultierenden Verbindungen der Formel (I) gegebenenfalls mit den entsprechenden (i) Lösemitteln und/oder (ii) Basen oder Säuren zu ihren Solvaten, Salzen und/oder Solvaten der Salze umsetzt.

Die Verbindung der Formel (II) ist literaturbekannt (R. Gompper, W. Toepfl, Chem. Ber. 1962, 95, 2861-2870). Die Verbindungen der Formeln (III) und (V) sind kommerziell erhältlich, literaturbekannt oder können in Analogie zu literaturbekannten Verfahren hergestellt werden (siehe z.B. H. Gielen, C. Alonso-Alija, M. Hendrix, U. Niewöhner, D. Schauß, Tetrahedron Lett. 2002, 43, 419-421).

Für den Verfahrensschritt (II) + (III) → (IV) eignen sich hochsiedende, inerte organische Lösemittel, die sich unter den Reaktionsbedingungen nicht verändern. Hierzu gehören bevorzugt Dimethylformamid, Dimethylsulfoxid oder Sulfolan. Ebenso ist es möglich, die Reaktion ohne Lösemittel in der Schmelze durchzuführen. Besonders bevorzugt ist eine Reaktionsführung ohne Lösemittel oder in Dimethylformamid.

Die Reaktion erfolgt im Allgemeinen in einem Temperaturbereich von +100°C bis +200°C, bevorzugt in einem Temperaturbereich von +125°C bis +150°C. Die Umsetzung kann bei normalem, erhöhtem oder bei erniedrigtem Druck durchgeführt werden (z.B. von 0.5 bis 5 bar). Im Allgemeinen arbeitet man bei Normaldruck.

Die Verbindung der Formel (III) wird hierbei in einer Menge von 1 bis 2 Mol, bevorzugt in einer äquivalenten Menge von 1 Mol, bezogen auf 1 Mol der Verbindung der Formel (II) eingesetzt.

Für den Verfahrensschritt (IV) + (V) → (I) eignen sich übliche organische Lösemittel, die sich unter den Reaktionsbedingungen nicht verändern. Hierzu gehören bevorzugt Dimethylformamid, Dimethylsulfoxid, Acetonitril, Dioxan oder Alkohole wie Methanol, Ethanol, Propanol, Isopropanol, n-Butanol oder tert.-Butanol. Ebenso ist es möglich, Gemische der genannten Lösemittel einzusetzen. Besonders bevorzugt ist Dimethylformamid oder Acetonitril.

Die Reaktion erfolgt im Allgemeinen in einem Temperaturbereich von +50°C bis +150°C, bevorzugt in einem Temperaturbereich von +70°C bis +100°C. Die Umsetzung kann bei normalem, erhöhtem oder bei erniedrigtem Druck durchgeführt werden (z.B. von 0.5 bis 5 bar). Im Allgemeinen arbeitet man bei Normaldruck.

Als Basen für den Verfahrensschritt (IV) + (V) → (I) eignen sich bevorzugt Alkalicarbonate wie Lithium-, Natrium-, Kalium- oder Cäsiumcarbonat oder organische Amin-Basen wie beispielsweise Pyridin, Triethylamin, Ethyldiisopropylamin, *N*-Methylmorpholin oder *N*-Methylpiperidin. Besonders bevorzugt ist Kaliumcarbonat.

Die Base wird hierbei in einer Menge von 1.5 bis 4 Mol, bevorzugt in einer Menge von 1.5 bis 2 Mol, bezogen auf 1 Mol der Verbindung der Formel (IV) eingesetzt. Die Verbindung der Formel (V) wird in einer Menge von 1 bis 1.5 Mol, bevorzugt in einer Menge von 1.2 Mol, bezogen auf 1 Mol der Verbindung der Formel (IV) eingesetzt.

Das erfindungsgemäße Verfahren kann durch das folgende Formelschema beispielhaft erläutert werden: 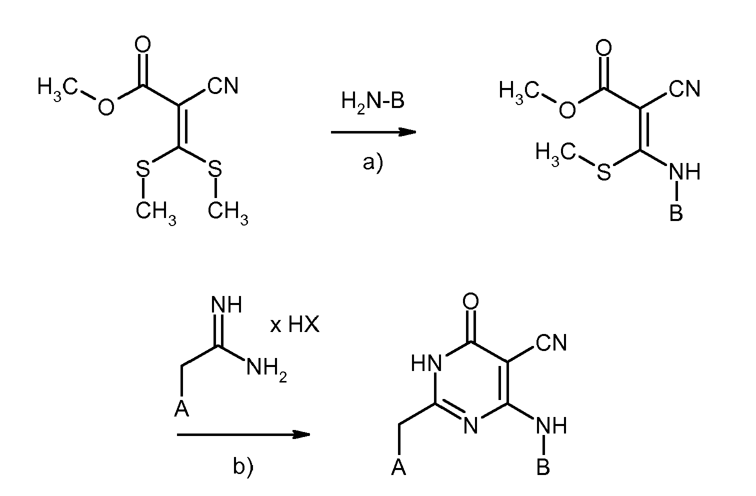 a) 150°C, 2 h; b) Kaliumcarbonat, DMF, 90°C, 16 h.

Die erfindungsgemäßen Verbindungen zeigen ein nicht vorhersehbares, wertvolles pharmakologisches Wirkspektrum. Sie zeichnen sich insbesondere durch eine Inhibition von PDE9A aus.

Überraschenderweise wurde gefunden, dass die erfindungsgemäßen Verbindungen zur Herstellung von Arzneimitteln zur Verbesserung der Wahrnehmung, Konzentrationsleistung, Lernleistung oder Gedächtnisleistung geeignet sind.

Die erfindungsgemäßen Verbindungen können aufgrund ihrer pharmakologischen Eigenschaften allein oder in Kombination mit anderen Arzneimitteln zur Verbesserung von Wahrnehmung, Konzentrationsleistung, Lern- und/oder Gedächtnisleistung eingesetzt werden.

Besonders eignen sich die erfindungsgemäßen Verbindungen zur Verbesserung der Wahrnehmung, Konzentrationsleistung, Lernleistung, oder Gedächtnisleistung nach kognitiven Störungen, wie sie insbesondere bei Situationen/Krankheiten/Syndromen auftreten wie "Mild cognitive impairment", Altersassoziierte Lern- und Gedächtnisstörungen, Altersassoziierte Gedächtnisverluste, Vaskuläre Demenz, Schädel-Hirn-Trauma, Schlaganfall, Demenz, die nach Schlaganfällen auftritt ("post stroke dementia"), post-traumatische Demenz, allgemeine Konzentrationsstörungen, Konzentrationsstörungen in Kindern mit Lern- und Gedächtnisproblemen, Alzheimer'sche Krankheit, Demenz mit Lewy-Körperchen, Demenz mit Degeneration der Frontallappen einschließlich des Pick's Syndroms, Parkinson'sche Krankheit, Progressive nuclear palsy, Demenz mit corticobasaler Degeneration, Amyotrope Lateralsklerose (ALS), Huntingtonsche Krankheit, Multiple Sklerose, Thalamische Degeneration, Creutzfeld-Jacob-Demenz, HIV-Demenz, Schizophrenie mit Demenz oder Korsakoff-Psychose.

Die *in* vitro-Wirkung der erfindungsgemäßen Verbindungen kann mit folgenden biologischen Assays gezeigt werden:

### PDE-Inhibition

Rekombinante PDE1C (GenBank/EMBL Accession Number: NM_005020, Loughney et al. J. Biol. Chem. 1996 271, 796-806), PDE2A (GenBank/EMBL Accession Number: NM_002599, Rosman et al. Gene 1997 191, 89-95), PDE3B (GenBank/EMBL Accession Number: NM_000922, Miki et al. Genomics 1996, 36, 476-485), PDE4B (GenBank/EMBL Accession Number: NM_002600, Obernolte et al. Gene. 1993, 129, 239-247), PDE5A (GenBank/EMBL Accession Number: NM_001083, Loughney et al. Gene 1998, 216, 139-147), PDE7B (GenBank/EMBL Accession Number: NM_018945, Hetman et al. Proc. Natl. Acad. Sci. U.S.A. 2000, 97, 472-476), PDE8A (GenBank/EMBL Accession Number: AF_056490, Fisher et al. Biochem. Biophys. Res. Commun. 1998 246, 570-577), PDE9A (Fisher et al., J. Biol. Chem, 1998, 273 (25): 15559 - 15564), E10A (GenBank/EMBL Accession Number: NM_06661, Fujishige et al. JBiol Chem. 1999, 274, 18438-45.), PDE11A (GenBank/EMBL Accession Number: NM_016953, Fawcett et al. Proc. Natl. Acad. Sci. 2000, 97, 3702-3707) wurden mit Hilfe des pFASTBAC Baculovirus Expressionssystems (GibcoBRL) in Sf9 Zellen exprimiert.

Die Testsubstanzen werden zur Bestimmung ihrer *in vitro* Wirkung an PDE 9A in 100 % DMSO aufgelöst und seriell verdünnt. Typischerweise werden Verdünnungsreihen von 200 µM bis 1.6 µM hergestellt (resultierende Endkonzentrationen im Test: 4 µM bis 0.032 µM). Jeweils 2 µL der verdünnten Substanzlösungen werden in die Vertiefungen von Mikrotiterplatten (Isoplate; Wallac Inc., Atlanta, GA) vorgelegt. Anschließend werden 50 µL einer Verdünnung des oben beschriebenen PDE9A Präparates hinzugefügt. Die Verdünnung des PDE9A Präparates wird so gewählt, dass während der späteren Inkubation weniger als 70% des Substrates umgesetzt wird (typische Verdünnung: 1: 10000; Verdünnungspuffer: 50 mM Tris/HCl pH 7.5, 8.3 mM MgCl₂, 1.7 mM EDTA, 0.2% BSA). Das Substrat, [8-³H] guanosine 3', 5'-cyclic phosphate (1 µCi/µL; Amersham Pharmacia Biotech., Piscataway, NJ) wird 1:2000 mit Assaypuffer (50 mM Tris/HCl pH 7.5, 8.3 mM MgCl₂, 1.7 mM EDTA) auf eine Konzentration von 0.0005µCi/µL verdünnt. Durch Zugabe von 50 µL (0.025 µCi) des verdünnten Substrates wird die Enzymreaktion schließlich gestartet. Die Testansätze werden für 60 min bei Raumtemperatur inkubiert und die Reaktion durch Zugabe von 25 µl eines in Assaypuffer gelösten PDE9A-Inhibitors (z.B. der Inhibitor aus Herstellbeispiel 1, 10 µM Endkonzentration) gestoppt. Direkt im Anschluss werden 25 µL einer Suspension mit 18 mg/mL Yttrium Scintillation Proximity Beads (Amersham Pharmacia Biotech., Piscataway, NJ.) hinzugefügt. Die Mikrotiterplatten werden mit einer Folie versiegelt und für 60 min bei Raumtemperatur stehengelassen. Anschließend werden die Platten für 30 s pro Vertiefung in einem Microbeta Szintillationzähler (Wallac Inc., Atlanta, GA) vermessen. IC₅₀-Werte werden anhand der graphischen Auftragung der Substanzkonzentration gegen die prozentuale Inhibition bestimmt.

Repräsentative Beispiele für die PDE9A-inhibierende Wirkung der erfindungsgemäßen Verbindungen sind anhand der IC₅₀-Werte in den Tabellen 1 und 2 aufgeführt:

**Tabelle 1: Inhibition von PDE-Isoenzymen durch Beispiel 3**

| Isoenzym | Species | IC₅₀ [nM] |
|---|---|---|
| PDE1C | human | > 4000 |
| PDE2A | human | > 4000 |
| PDE3B | human | > 4000 |
| PDE4B | human | > 4000 |
| PDE5A | human | 1400 |
| PDE7B | human | > 4000 |
| PDE8A | human | > 4000 |
| PDE9A | human | 52 |
| PDE10A | human | > 4000 |

**Tabelle 2: PDE9A-inhibierende Wirkung erfindungsgemäßer Verbindungen**

| Beispiel | IC₅₀ [nM] |
|---|---|
| 1 | 75 |
| 3 | 52 |
| 7 | 54 |
| 14 | 75 |
| 23 | 87 |

Die *in vitro* Wirkung von Testsubstanzen an rekombinanter PDE3B, PDE4B, PDE7B, PDE8A, PDE10A und PDE11A wird nach dem oben für PDE 9A beschriebenen Testprotokoll mit folgenden Anpassungen bestimmt: Als Substrat wird [5',8-³H] adenosine 3', 5'-cyclic phosphate (1 µCi/µL; Amersham Pharmacia Biotech., Piscataway, NJ) verwendet. Die Zugabe einer Inhibitorlösung zum Stoppen der Reaktion ist nicht notwendig. Stattdessen wird in Anschluss an die Inkubation von Substrat und PDE direkt mit der Zugabe der Yttrium Scintillation Proximity Beads wie oben beschrieben fortgefahren und dadurch die Reaktion gestoppt. Für die Bestimmung einer entsprechenden Wirkung an rekombinanter PDE1C, PDE2A und PDE5A wird das Protokoll zusätzlich wie folgt angepasst: Bei PDE1C werden zusätzlich Calmodulin 10⁻⁷ M und CaCl₂ 3mM zum Reaktionsansatz gegeben. PDE2A wird im Test durch Zugabe von cGMP 1 µM stimuliert und mit einer BSA Konzentration von 0,01 % getestet. Für PDE1C und PDE2A wird als Substrat [5',8-³H] adenosine 3', 5'-cyclic phosphate (1 µCi/µL; Amersham Pharmacia Biotech., Piscataway, NJ), für PDE5A [8-³H] guanosine 3', 5'-cyclic phosphate (1 µCi/µL; Amersham Pharmacia Biotech., Piscataway, NJ) eingesetzt.

### Langzeitpotenzierung

Langzeitpotenzierung wird als ein zelluläres Korrelat für Lern- und Gedächtnisvorgänge angesehen. Zur Bestimmung, ob PDE9 Inhibition einen Einfluss auf Langzeitpotenzierung hat, kann folgende Methode angewandt werden:

Rattenhippokampi werden in einen Winkel von etwa 70 Grad im Verhältnis zur Schnittklinge plaziert (Chopper). In Abständen von 400 µm wird der Hippokampus zerschnitten. Die Schnitte werden mit Hilfe eines sehr weichen, stark benetzten Pinsels (Marderhaar) von der Klinge genommen und in ein Glasgefäß mit carbogenisierter gekühlter Nährlösung (124 mM NaCl, 4,9 mM KCl, 1,3 mM MgSO₄* 7H₂O, 2,5 mM CaCl²⁺ Wasser- frei, 1,2 mM KH₂PO₄ 25,6 mM NaHCO₃ 10 mM Glucose, pH 7.4) überführt. Während der Messung befinden sich die Schnitte in einer temperierten Kammer unter einem Flüssigkeitsspiegel von 1-3 mm Höhe. Die Durchflussrate beträgt 2,5 ml/min. Die Vorbegasung erfolgt unter geringen Überdruck (etwa 1 atm) sowie über eine Mikrokanüle in der Vorkammer. Die Schnittkammer ist mit der Vorkammer so verbunden, dass eine Minizirkulation aufrechterhalten werden kann. Als Antrieb der Minizirkulation wird das durch die Mikrokanüle ausströmende Carbogen eingesetzt. Die frisch präparierten Hippokampusschnitte werden mindestens 1 Stunde bei 33°C in der Schnittkammer adaptiert.

Die Reizstärke wird so gewählt, dass die fokalen exzitatiorischen postsynaptischen Potentiale (fEPSP) 30 % des maximalen exzitatorischen postsynaptischen Potentials (EPSP) betragen. Mit Hilfe einer monopolaren Stimulationselektrode, die aus lackiertem Edelstahl besteht und eines stromkonstanten, biphasischen Reizgenerators (AM-Systems 2100), werden lokal die Schaffer-Kollateralen erregt (Spannung: 1-5 V, Impulsbreite einer Polarität 0,1 ms, Gesamtimpuls 0,2 ms).

Mit Hilfe von Glaselektroden (Borosilikatglas mit Filament, 1-5 MOhm, Durchmesser: 1,5 mm, Spitzendurchmesser: 3-20 µm), die mit normaler Nährlösung gefüllt sind, werden aus dem Stratum radiatum die exzitatorischen postsynaptischen Potentiale (fEPSP) registriert. Die Messung der Feldpotentiale geschieht gegenüber einer chlorierten Referenzelektrode aus Silber, die sich am Rande der Schnittkammer befindet, mit Hilfe eines Gleichspannungsverstärkers. Das Filtern der Feldpotentiale erfolgt über einen Low-Pass Filter (5 kHz). Für die statistische Analyse der Experimente wird der Anstieg (slope) der fEPSPs (fEPSP-Anstieg) ermittelt. Die Aufnahme, Analyse und Steuerung des Experimentes erfolgt mit Hilfe eines Softwareprogrammes (PWIN), welches in der Abteilung Neurophysiologie entwickelt worden ist. Die Mittelwertbildung der fEPSP-Anstiegswerte zu den jeweiligen Zeitpunkten und die Konstruktion der Diagramme erfolgt mit Hilfe der Software EXCEL, wobei ein entsprechendes Makro die Aufnahme der Daten automatisiert.

Superfusion der Hippokampusschnitte mit einer 10 µM Lösung der erfindungsgemäßen Verbindungen führt zu einer signifikanten Steigerung der LTP.

Die in vivo-Wirkung der erfindungsgemäßen Verbindungen kann zum Beispiel wie folgt gezeigt werden:

### Sozialer Wiedererkennungstest

Der Soziale Wiedererkennungstest ist ein Lern- und Gedächtnistest. Er misst die Fähigkeit von Ratten, zwischen bekannten und unbekannten Artgenossen zu unterscheiden. Deshalb eignet sich dieser Test zur Prüfung der lern- oder gedächtnisverbessernden Wirkung der erfindungsgemäßen Verbindungen.

Adulte Ratten, die in Gruppen gehalten werden, werden 30 min vor Testbeginn einzeln in Testkäfige gesetzt. Vier min vor Testbeginn wird das Testtier in eine Beobachtungsbox gebracht. Nach dieser Adaptationszeit wird ein juveniles Tier zu dem Testtier gesetzt und 2 min lang die absolute Zeit gemessen, die das adulte Tier das Junge inspiziert (Trial 1). Gemessen werden alle deutlich auf das Jungtier gerichteten Verhaltensweisen, d.h. ano-genitale Inspektion, Verfolgen sowie Fellpflege, bei denen das Alttier einen Abstand von höchstens 1 cm zu dem Jungtier hatte. Danach wird das Juvenile herausgenommen, das Adulte mit einer erfindungsgemäßen Verbindung oder Vehikel behandelt und anschließend in seinen Heimkäfig zurückgesetzt. Nach einer Retentionszeit von 24 Stunden wird der Test wiederholt (Trial 2). Eine verringerte Soziale Interaktionszeit im Vergleich zu Trial 1 zeigt an, dass die adulte Ratte sich an das Jungtier erinnert.

Die adulten Tiere werden entweder in einem festgelegten Zeitabstand (z.B. 1 Stunde) vor Trial 1 oder direkt im Anschluss an Trial 1 entweder mit Vehikel (10 % Ethanol, 20 % Solutol, 70 % physiologische Kochsalzlösung) oder 0,1 mg/kg, 0,3 mg/kg, 1,0 mg/kg bzw. 3,0 mg/kg erfindungsgemäßer Verbindung, gelöst in 10 % Ethanol, 20 % Solutol, 70 % physiologische Kochsalzlösung intraperitoneal injiziert. Vehikel behandelte Ratten zeigen keine Reduktion der sozialen Interaktionszeit in Trial 2 verglichen mit Trial 1. Sie haben folglich vergessen, dass sie schon einmal Kontakt mit dem Jungtier hatten. Überraschenderweise ist die soziale Interaktionszeit im zweiten Durchgang nach Behandlung mit den erfindungsgemäßen Verbindungen signifikant gegenüber den Vehikel behandelten reduziert. Dies bedeutet, dass die substanzbehandelten Ratten sich an das juvenile Tier erinnert haben und somit die erfindungsgemäßen Verbindungen eine verbessernde Wirkung auf Lernen und Gedächtnis aufweist.

Die neuen Wirkstoffe können in bekannter Weise in die üblichen Formulierungen überführt werden, wie Tabletten, Dragees, Pillen, Granulate, Aerosole, Sirupe, Emulsionen, Suspensionen und Lösungen, unter Verwendung inerter, nicht toxischer, pharmazeutisch geeigneter Trägerstoffe oder Lösungsmittel. Hierbei soll die therapeutisch wirksame Verbindung jeweils in einer Konzentration von etwa 0,5 bis 90 Gew.-% der Gesamtmischung vorhanden sein, d.h. in Mengen, die ausreichend sind, um den angegebenen Dosierungsspielraum zu erreichen.

Die Formulierungen werden beispielsweise durch Verstrecken der Wirkstoffe mit Lösungsmitteln und/oder Trägerstoffen, gegebenenfalls unter Verwendung von Emulgiermitteln und/oder Dispergiermitteln hergestellt, wobei z.B. im Fall der Benutzung von Wasser als Verdünnungsmittel gegebenenfalls organische Lösungsmittel als Hilfslösungsmittel verwendet werden können.

Die Applikation erfolgt in üblicher Weise, vorzugsweise oral, transdermal oder parenteral, insbesondere perlingual oder intravenös. Sie kann aber auch durch Inhalation über Mund oder Nase, beispielsweise mit Hilfe eines Sprays, oder topisch über die Haut erfolgen.

Im Allgemeinen hat es sich als vorteilhaft erwiesen, Mengen von etwa 0,001 bis 10, bei oraler Anwendung vorzugsweise etwa 0,005 bis 3 mg/kg Körpergewicht zur Erzielung wirksamer Ergebnisse zu verabreichen.

Trotzdem kann es gegebenenfalls erforderlich sein, von den genannten Mengen abzuweichen, und zwar in Abhängigkeit vom Körpergewicht bzw. der Art des Applikationsweges, vom individuellen Verhalten gegenüber dem Medikament, der Art von dessen Formulierung und dem Zeitpunkt bzw. Intervall, zu welchen die Verabreichung erfolgt. So kann es in einigen Fällen ausreichend sein, mit weniger als der vorgenannten Mindestmenge auszukommen, während in anderen Fällen die genannte obere Grenze überschritten werden muss. Im Falle der Applikation größerer Mengen kann es empfehlenswert sein, diese in mehreren Einzelgaben über den Tag zu verteilen.

Soweit nicht anders angegeben, beziehen sich alle Mengenangaben auf Gewichtsprozente. Lösungsmittelverhältnisse, Verdünnungsverhältnisse und Konzentrationsangaben von flüssig/flüssig-Lösungen beziehen sich jeweils auf das Volumen. Die Angabe "w/v" bedeutet "weight/volume" (Gewicht/Volumen). So bedeutet beispielsweise "10 % w/v": 100 ml Lösung oder Suspension enthalten 10 g Substanz.

**Abkürzungen:**

| | |
|---|---|
| DMF | *N,N* Dimethylformamid |
| DMSO | Dimethylsulfoxid |
| d. Th. | der Theorie (bei Ausbeute) |
| ESI | Elektrospray-Ionisation (bei MS) |
| h | Stunde(n) |
| HPLC | Hochdruck-, Hochleistungsflüssigchromatographie |
| LC-MS | Flüssigchromatographie-gekoppelte Massenspektroskopie |
| min | Minuten |
| MS | Massenspektroskopie |
| NMR | Kernresonanzspektroskopie |
| RT | Raumtemperatur |
| Rₜ | Retentionszeit (bei HPLC) |
| THF | Tetrahydrofuran |

### HPLC- und LC-MS-Methoden:

### Methode 1:

Instrument: Micromass Quattro LCZ, mit HPLC Agilent Serie 1100; Säule: Grom-Sil 120 ODS-4 HE, 50 mm x 2.0 mm, 3 µm; Eluent A: 1 1 Wasser + 1 ml 50%-ige Ameisensäure, Eluent B: 1 1 Acetonitril + 1 ml 50%-ige Ameisensäure; Gradient: 0.0 min 100% A → 0.2 min 100% A → 2.9 min 30% A → 3.1 min 10% A → 4.5 min 10% A; Ofen: 55°C; Fluss: 0.8 ml/min; UV-Detektion: 208-400 nm.

### Methode 2:

Gerätetyp MS: Micromass ZQ; Gerätetyp HPLC: Waters Alliance 2795; Säule: Phenomenex Synergi 2µ Hydro-RP Mercury 20 mm x 4 mm; Eluent A: 1 1 Wasser + 0.5 ml 50%-ige Ameisensäure, Eluent B: 1 1 Acetonitril + 0.5 ml 50%-ige Ameisensäure; Gradient: 0.0 min 90% A Fluss 1 ml/min → 2.5 min 30% A Fluss 2 ml/min → 3.0 min 5% A Fluss 2 ml/min → 4.5 min 5% A Fluss 2 ml/min; Ofen: 50°C; UV-Detektion: 210 nm.

### Methode 3:

Gerätetyp MS: Micromass ZQ; Gerätetyp HPLC: HP 1100 Series; UV DAD; Säule: Grom-Sil 120 ODS-4 HE 50 mm x 2 mm, 3.0 µm; Eluent A: Wasser + 500 µl 50%-ige Ameisensäure / 1, Eluent B: Acetonitril + 500 µl 50%-ige Ameisensäure / 1; Gradient: 0.0 min 0% B → 2.9 min 70% B → 3.1 min 90% B → 4.5 min 90% B; Ofen: 50°C; Fluss: 0.8 ml/min; UV-Detektion: 210 nm.

### Methode 4:

Gerätetyp MS: Micromass ZQ; Gerätetyp HPLC: Waters Alliance 2795; Säule: Merck Chromolith SpeedROD RP-18e 50 mm x 4.6 mm; Eluent A: Wasser + 500 µl 50%-ige Ameisensäure / 1, Eluent B: Acetonitril + 500 µl 50%-ige Ameisensäure / 1; Gradient: 0.0 min 10% B → 3.0 min 95% B → 4.0 min 95% B; Ofen: 35°C; Fluss: 0.0 min 1.0 ml/min → 3.0 min 3.0 ml/min → 4.0 min 3.0 ml/min; UV-Detektion: 210 nm.

### Methode 5:

Gerätetyp MS: Micromass ZQ; Gerätetyp HPLC: HP 1100 Series; UV DAD; Säule: Phenomenex Synergi 2µ Hydro-RP Mercury 20 mm x 4 mm; Eluent A: 1 1 Wasser + 0.5 ml 50%-ige Ameisensäure, Eluent B: 1 1 Acetonitril + 0.5 ml 50%-ige Ameisensäure; Gradient: 0.0 min 90% A Fluss 1 ml/min → 2.5 min 30% A Fluss 2 ml/min → 3.0 min 5% A Fluss 2 ml/min → 4.5 min 5% A Fluss 2 ml/min; Ofen: 50°C; UV-Detektion: 210 nm.

### Methode 6:

Instrument: Micromass Quattro LCZ mit HPLC Agilent Serie 1100; Säule: Phenomenex Synergi 2µ Hydro-RP Mercury 20 mm x 4 mm; Eluent A: 11 Wasser + 0.5 ml 50%-ige Ameisensäure, Eluent B: 11 Acetonitril + 0.5 ml 50%-ige Ameisensäure; Gradient: 0.0 min 90% A Fluss 1 ml/min → 2.5 min 30% A Fluss 2 ml/min → 3.0 min 5% A Fluss 2 ml/min → 4.5 min 5% A Fluss 2 ml/min; Ofen: 50°C; UV-Detektion: 208-400 nm.

### Methode 7:

Gerätetyp MS: Micromass ZQ; Gerätetyp HPLC: Waters Alliance 2790; Säule: Grom-Sil 120 ODS-4 HE 50 mm x 2 mm, 3.0 µm; Eluent A: Wasser + 500 µl 50%-ige Ameisensäure / 1, Eluent B: Acetonitril + 500 µl 50%-ige Ameisensäure / 1; Gradient: 0.0 min 5% B → 2.0 min 40% B → 4.5 min 90% B → 5.5 min 90% B; Ofen: 45°C; Fluss: 0.0 min 0.75 ml/min → 4.5 min 0.75 ml/min → 5.5 min 1.25 ml/min; UV-Detektion: 210 nm.

### Methode 8:

Instrument: HP 1100 mit DAD-Detektion; Säule: Kromasil RP-18, 60 mm x 2 mm, 3.5 µm; Eluent A: 5 ml HClO₄/H₂O, Eluent B: Acetonitril; Gradient: 0 min 2% B → 0.5 min 2% B → 4.5 min 90% B → 6.5 min 90% B; Fluss: 0.75 ml/min; Temperatur: 30°C; UV-Detektion 210 nm.

### Methode 9:

Instrument: Micromass Platform LCZ mit HPLC Agilent Serie 1100; Säule: Phenomenex Synergi 2µ Hydro-RP Mercury 20 mm x 4 mm; Eluent A: 1 1 Wasser + 0.5 ml 50%-ige Ameisensäure, Eluent B: 1 1 Acetonitril + 0.5 ml 50%-ige Ameisensäure; Gradient: 0.0 min 90% A Fluss 1 ml/min → 2.5 min 30% A Fluss 2 ml/min → 3.0 min 5% A Fluss 2 ml/min → 4.5 min 5% A Fluss 2 ml/min; Ofen: 50°C; UV-Detektion: 210 nm.

### Methode 10:

Gerätetyp MS: Micromass ZQ; Gerätetyp HPLC: Waters Alliance 2790; Säule: Grom-Sil 120 ODS-4 HE 50 mm x 2 mm, 3.0 µm; Eluent A: Wasser + 500 µl 50%-ige Ameisensäure / 1, Eluent B: Acetonitril + 500 µl 50%-ige Ameisensäure / 1; Gradient: 0.0 min 0% B → 0.2 min 0% B → 2.9 min 70% B → 3.1 min 90% B → 4.5 min 90% B; Ofen: 45°C; Fluss: 0.8 ml/min; UV-Detektion: 210 nm.

### Ausgangsverbindungen:

### Beispiel 1A

### 2-Cyclopentylethanamidin-Hydrochlorid

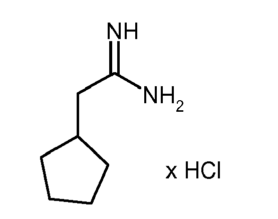

In einer Argonatmosphäre werden 58.2 g (1.09 mol) Ammoniumchlorid in 350 ml Toluol suspendiert und auf 0°C gekühlt. Es werden 544 ml einer 2 M Lösung von Trimethylaluminium in Toluol zugetropft und das Gemisch im Anschluss 2 h bei RT gerührt. Dann erfolgt Zugabe von 34 g (217 mmol) Ethylcyclopentylacetat. Anschließend wird über Nacht bei 80°C gerührt. Nach dem Abkühlen auf 0°C werden 400 ml Methanol zugetropft, dann der entstandene Feststoff abgesaugt. Dieser wird noch mehrmals gut mit Methanol gewaschen, und die vereinigten Filtrate werden im Vakuum eingeengt. Der Rückstand wird in Dichlormethan/Methanol 10:1 suspendiert und wiederum der unlösliche Feststoff abgetrennt. Das nun gewonnene Filtrat wird eingeengt und ergibt 23 g (65% d. Th.) des gewünschten Produkts.
MS (ESIpos): m/z = 127 [M+H]⁺ (freie Base).

Analog zu Beispiel 1A werden 2-Cyclohexylethanamidin-Hydrochlorid und 3-Methylpentanamidin-Hydrochlorid aus den jeweiligen Estern in 56%-iger bzw. 61%-iger Ausbeute hergestellt (siehe auch H. Gielen, C. Alonso-Alija, M. Hendrix, U. Niewöhner, D. Schauß, Tetrahedron Lett. 2002, 43, 419-421).

### Beispiel 2A

### Methyl 2-cyano-3-[(4-fluorphenyl)amino]-3-(methylsulfanyl)-2-propenoat

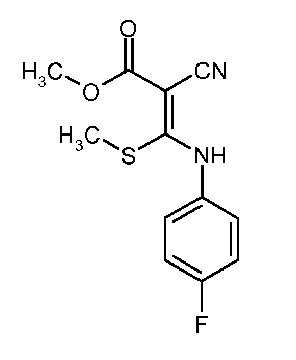

0.5 g (4.5 mmol) 4-Fluoranilin werden mit 0.91 g (4.5 mmol) Methyl 3,3-bis-(methylthio)-2-cyanoacrylat (R. Gompper, W. Toepfl, Chem. Ber. 1962, 95, 2861-2870) gut vermischt. Die Reaktionsmischung wird 2 h auf 150°C erhitzt, wobei eine Schmelze entsteht. Nach dem Abkühlen wird ein heller Feststoff erhalten, der mehrfach mit Methanol gewaschen wird. Man erhält 0.68 g (55.7% d. Th.) des gewünschten Produkts.
LC-MS (Methode 1): Rₜ = 2.6 min.
MS (ESIpos): m/z = 267 [M+H]⁺.

### Beispiel 3A

### Methyl 2-cyano-3-[(4-methyl-3-pyridinyl)amino]-3-(methylsulfanyl)-2-propenoat

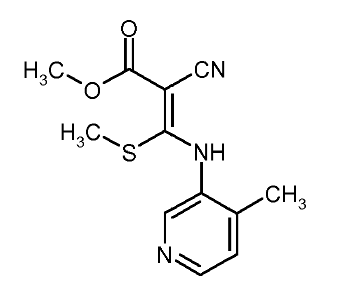

Analog zur Herstellung von Beispiel 2A werden aus 2.0 g (18.49 mmol) 3-Amino-4-methylpyridin und 3.76 g (18.49 mmol) Methyl 3,3-bis(methylthio)-2-cyanoacrylat 1.4 g (29.6% d. Th.) der Titelverbindung als Feststoff erhalten.

### Beispiel 4A

### Methyl 2-cyano-3-[(3-fluorphenyl)amino]-3-(methylsulfanyl)-2-propenoat

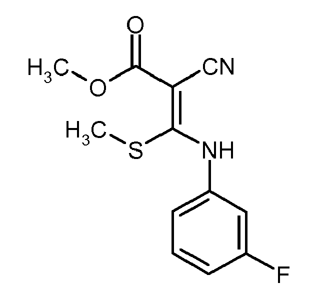

Analog zur Herstellung von Beispiel 2A werden aus 0.5 g (4.5 mmol) 3-Fluoranilin und 0.91 g (4.5 mmol) Methyl 3,3-bis(methylthio)-2-cyanoacrylat 0.43 g (36% d. Th.) der Titelverbindung als Feststoff erhalten.
LC-MS (Methode 1): Rₜ = 2.63 min.
MS (ESIpos): m/z = 267 [M+H]⁺.

### Beispiel 5A

### Methyl 2-cyano-3-[(3-chlorphenyl)amino]-3-(methylsulfanyl)-2-propenoat

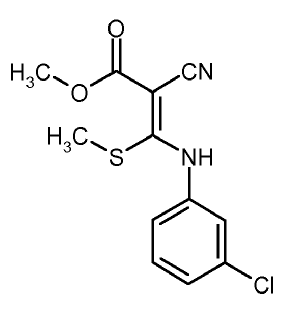

Analog zur Herstellung von Beispiel 2A werden aus 0.5 g (3.9 mmol) 3-Chloranilin und 0.79 g (3.9 mmol) Methyl 3,3-bis(methylthio)-2-cyanoacrylat 0.53 g (48% d. Th.) der Titelverbindung als Feststoff erhalten.
LC-MS (Methode 1): Rₜ = 2.78 min.
MS (ESIpos): m/z = 283 [M+H]⁺.

### Beispiel 6A

### Methyl 2-cyano-3-[(3-methoxyphenyl)amino]-3-(methylsulfanyl)-2-propenoat

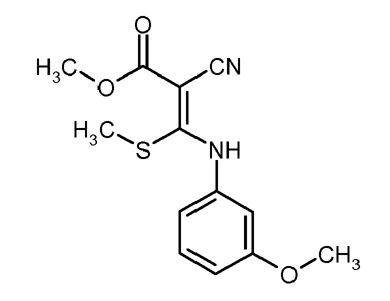

Analog zur Herstellung von Beispiel 2A werden aus 0.5 g (4.0 mmol) 3-Methoxyanilin und 0.8 g (4.0 mmol) Methyl 3,3-bis(methylthio)-2-cyanoacrylat 0.47 g (41% d. Th.) der Titelverbindung als Feststoff erhalten.
LC-MS (Methode 1): Rₜ = 2.63 min.
MS (ESIpos): m/z = 279 [M+H]⁺.

### Beispiel 7A

### Methyl 2-cyano-3-[(3-fluor-2-methylphenyl)amino]-3-(methylsulfanyl)-2-propenoat

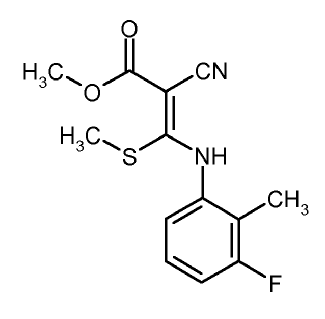

Analog zur Herstellung von Beispiel 2A werden aus 0.2 g (1.59 mmol) 3-Fluor-2-methylanilin und 0.32 g (1.59 mmol) Methyl 3,3-bis(methylthio)-2-cyanoacrylat 0.15 g (34% d. Th.) der Titelverbindung als Feststoff erhalten.
LC-MS (Methode 6): Rₜ = 2.5 min.
MS (ESIpos): m/z = 281 [M+H]⁺.
**Beispiel 8A**

### Methyl 2-cyano-3-[(2,5-dimethylphenyl)amino]-3-(methylsulfanyl)-2-propenoat

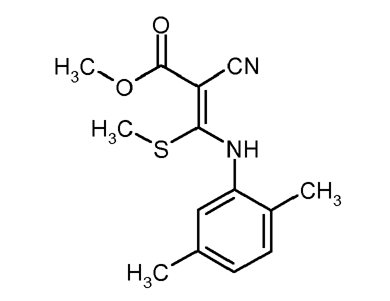

Analog zur Herstellung von Beispiel 2A werden aus 0.54 g (4.4 mmol) 2,5-Dimethylanilin und 0.9 g (4.4 mmol) Methyl 3,3-bis(methylthio)-2-cyanoacrylat 0.9 g (75% d. Th.) der Titelverbindung als Feststoff erhalten.
LC-MS (Methode 1): Rₜ = 2.91 min.
MS (ESIpos): m/z = 277 [M+H]⁺.

### Beispiel 9A

### Methyl 2-cyano-3-[(2-methoxyphenyl)amino]-3-(methylsulfanyl)-2-propenoat

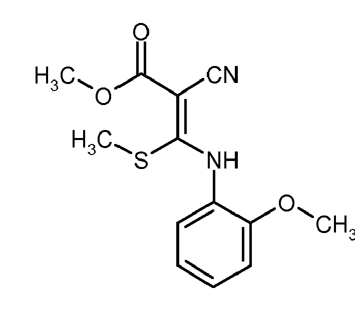

Analog zur Herstellung von Beispiel 2A werden aus 0.6 g (5.0 mmol) 2-Methoxyanilin und 1.0 g (5.0 mmol) Methyl 3,3-bis(methylthio)-2-cyanoacrylat 0.9 g (67% d. Th.) der Titelverbindung als Feststoff erhalten.
LC-MS (Methode 7): Rₜ = 3.01 min.
MS (ESIpos): m/z = 279 [M+H]⁺
¹H-NMR (300 MHz, DMSO-d₆): δ = 2.28 (s, 3H), 3.71 (s, 3H), 3.84 (s, 3H), 7.00 (m, 1H), 7.17 (m, 1H), 7.33 (m, 1H), 7.41 (m, 1H).

### Beispiel 10A

### Methyl 2-cyano-3-[(4-fluor-2-methylphenyl)amino]-3-(methylsulfanyl)-2-propenoat

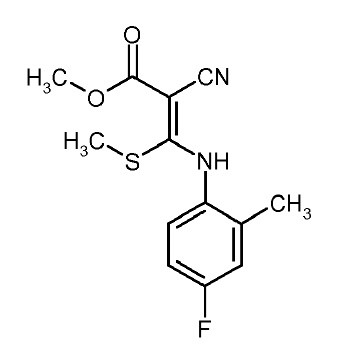

Analog zur Herstellung von Beispiel 2A werden aus 0.62 g (5.0 mmol) 2-Methyl-4-fluoranilin und 1.01 g (5.0 mmol) Methyl 3,3-bis(methylthio)-2-cyanoacrylat 0.7 g (50% d. Th.) der Titelverbindung als Feststoff erhalten.
LC-MS (Methode 4): Rₜ = 2.28 min.
MS (ESIpos): m/z = 281 [M+H]⁺
¹H-NMR (300 MHz, DMSO-d₆): δ = 2.22 (s, 3H), 2.27 (s, 3H), 3.70 (s, 3H), 7.11 (m, 1H), 7.23 (m, 1H), 7.33 (m, 1H), 7.34 (m, 1H).

### Beispiel 11A

### Methyl 2-cyano-3-[(2-methylphenyl)amino]-3-(methylsulfanyl)-2-propenoat

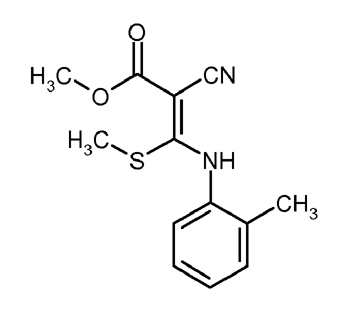

Analog zur Herstellung von Beispiel 2A werden aus 1.6 g (15.0 mmol) 2-Methylanilin und 3.01 g (15.0 mmol) Methyl 3,3-bis(methylthio)-2-cyanoacrylat 2.5 g (63% d. Th.) der Titelverbindung als Feststoff erhalten.
LC-MS (Methode 7): Rₜ = 3.08 min.
MS (ESIpos): m/z = 263 [M+H]⁺
¹H-NMR (300 MHz, DMSO-d₆): δ = 2.27 (s, 3H), 2.23 (s, 3H), 3.70 (s, 3H), 7.28 (m, 4H).

### Beispiel 12A

### Methyl 2-cyano-3-(methylsulfanyl)-3-[(2-propylphenyl)amino]-2-propenoat

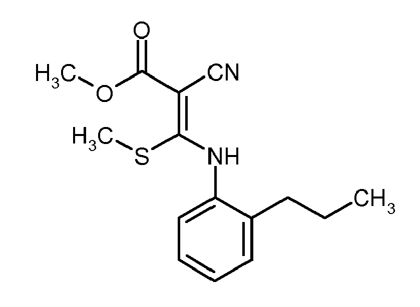

Analog zur Herstellung von Beispiel 2A werden aus 0.5 g (3.7 mmol) 2-Propylanilin und 0.7 g (3.7 mmol) Methyl 3,3-bis(methylthio)-2-cyanoacrylat 0.7 g (61% d. Th.) der Titelverbindung als Feststoff erhalten.
LC-MS (Methode 3): Rₜ = 3.52 min.
MS (ESIpos): m/z = 291 [M+H]⁺.

### Beispiel 13A

### Methyl 2-cyano-3-(methylsulfanyl)-3-(3-pyridinylamino)-2-propenoat

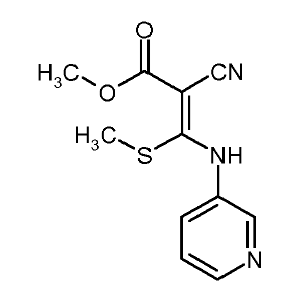

1.39 g (14.8 mmol) 3-Aminopyridin werden in 50 ml THF gelöst, auf -20°C abgekühlt und mit 7.4 ml einer 2 M Lösung von n-Buthyllithium in Hexan versetzt. Es wird 15 min. gerührt, dann erfolgt Zugabe von 2.00 g (9.84 mmol) Methyl 3,3-bis(methylthio)-2-cyanoacrylat. Unter Rühren wird der Ansatz auf Raumtemperatur erwärmt und anschließend mit Eiswasser hydrolysiert. Das Produkt wird mit Dichlormethan extrahiert. Nach Trocknung über Natriumsulfat wird das Lösungsmittel im Vakuum entfernt und der Rückstand mittels präparativer HPLC gereinigt. Man erhält 0.44 g (18.1% d. Th.) des gewünschten Produkts.
HPLC (Methode 8): Rₜ = 3.06 min.
MS (ESIpos): m/z = 250 [M+H]⁺, 272 [M+Na]⁺.

### Beispiel 14A

### (3S)-3-Methylpentannitril

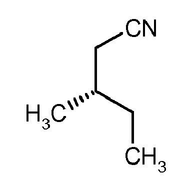

5 g (29.78 mmol) (2*S*)-2-Methylbutylmethansulfonat werden in 15 ml Dimethylformamid mit 1.5 g (44.66 mmol) Natriumcyanid über Nacht auf 80°C erhitzt. Nach Abkühlen auf Raumtemperatur wird mit 150 ml Wasser verdünnt und fünfmal mit Diethylether extrahiert. Die vereinigten organischen Phasen werden mit Wasser und mit gesättigter Natriumchlorid-Lösung gewaschen. Nach Trocknen über Natriumsulfat wird das Lösungsmittel bei Raumtemperatur im Vakuum entfernt. Es werden 2.3 g (67% d. Th.) Rohprodukt erhalten, das ohne weitere Reinigung in der nächsten Stufe eingesetzt wird.
¹H-NMR (200 MHz, DMSO-d₆): δ = 0.87 (t, 3H), 0.92 (d, 3H), 1.30 (m, 2H), 1.67 (m, 1H), 2.42 (dd, 2H).

### Beispiel 15A

### (3S)-3-Methylpentanamidin-Hydrochlorid

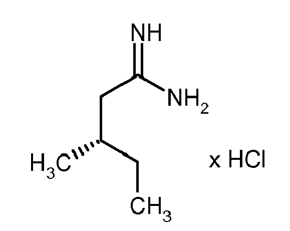

In einer Argonatmosphäre werden 1.1 g (20.5 mmol) Ammoniumchlorid in 20 ml Toluol suspendiert und auf 0°C gekühlt. Es werden 10.29 ml einer 2 M Lösung von Trimethylaluminium in Toluol zugetropft und das Gemisch im Anschluss 2 h bei RT gerührt. Dann erfolgt Zugabe von 1 g (10.29 mmol) (3*S*)-3-Methylpentannitril. Anschließend wird 2 Tage lang bei 80°C gerührt. Nach Abkühlen auf 0°C werden 40 ml Methanol zugetropft und dann der entstandene Feststoff abgesaugt. Dieser wird noch mehrmals gut mit Methanol gewaschen, und die vereinigten Filtrate werden im Vakuum eingeengt. Der Rückstand wird in Dichlormethan/Methanol 10:1 suspendiert und wiederum der unlösliche Feststoff abgetrennt. Das nun gewonnene Filtrat wird eingeengt und ergibt 1.01 g (64% d. Th.) des gewünschten Produkts.
LC-MS (Methode 2): Rₜ = 0.31 min.
MS (ESIpos): m/z = 115 [M+H]⁺ (freie Base).

### Beispiel 16A

### Methyl 2-cyano-3-[(5-fluor-2-methylphenyl)amino]-3-(methylsulfanyl)-2-propenoat

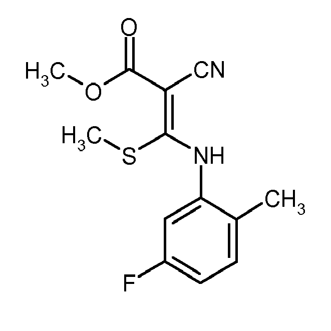

Analog zur Herstellung von Beispiel 2A werden aus 1.5 g (11.98 mmol) 5-Fluor-2-methylanilin und 2.4 g (11.98 mmol) Methyl 3,3-bis(methylthio)-2-cyanoacrylat 1.7 g (52% d. Th.) der Titelverbindung als Feststoff erhalten.
LC-MS (Methode 6): Rₜ = 2.49 min.
MS (ESIpos): m/z = 281 [M+H]⁺.

### Ausführungsbeispiele:

### Beispiel 1

### 2-(Cyclopentylmethyl)-4-[(4-fluorphenyl)amino]-6-oxo-1,6-dihydro-5-pyrimidincarbonitril

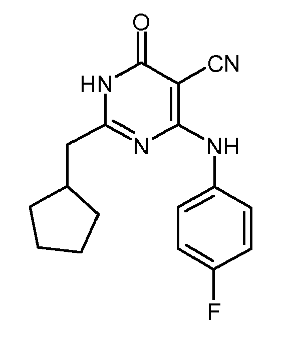

0.1 g (0.37 mmol) Methyl 2-cyano-3-[(4-fluorphenyl)amino]-3-(methylsulfanyl)-2-propenoat, 0.07 g (0.41 mmol) 2-Cyclopentylethanamidin-Hydrochlorid und 0.11 g (0.82 mmol) Kaliumcarbonat werden über Nacht in 1 ml Dimethylformamid auf 90°C erhitzt. Nach Filtration wird das Filtrat mit konzentrierter Salzsäure angesäuert, wobei das Produkt ausfällt. Nach mehrfachem Waschen mit Wasser und Trocknen im Hochvakuum werden 54 mg (46% d. Th.) des Produkts als farbloser Feststoff erhalten.
LC-MS (Methode 1): Rₜ = 2.86 min.
MS (ESIpos): m/z = 313 [M+H]⁺
¹H-NMR (300 MHz, DMSO-d₆): δ = 1.13 (m, 2H), 1.56 (m, 6H), 2.15 (m, 1H), 2.44 (d, 2H), 7.15 (dd, 2H), 7.41 (dd, 2H), 9.66 (s, 1H), 12.36 (s, 1H).

### Beispiel 2

### 2-(Cyclopentylmethyl)-4-[(4-methyl-3-pyridinyl)amino]-6-oxo-1,6-dihydro-5-pyrimidincarbonitril

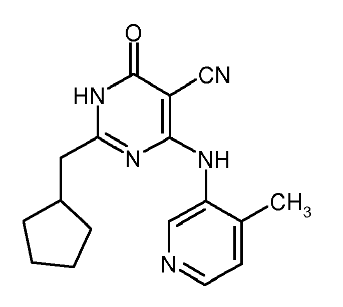

0.2 g (0.76 mmol) Methyl 2-cyano-3-[(4-methyl-3-pyridinyl)amino]-3-(methylsulfanyl)-2-propenoat werden mit 0.13 g (0.85 mmol) 2-Cyclopentylethanamidin-Hydrochlorid und 0.23 g (1.67 mmol) Kaliumcarbonat in 4 ml DMF gelöst und drei Tage lang bei 90°C gerührt. Nach dem Abkühlen wird das Produkt mittels präparativer HPLC gereinigt (YMC Gel ODS-AQ S 5/15 µm; Eluent A: Wasser, Eluent B: Acetonitril; Gradient: 0 min 30% B, 5 min 30% B, 50 min 95% B) gereinigt. Es werden 60 mg (25% d. Th.) des Produkts erhalten.
LC-MS (Methode 5): Rₜ = 1.57 min.
MS (ESIpos): m/z = 310 [M+H]⁺
¹H-NMR (300 MHz, DMSO-d₆): δ = 1.04 (m, 2H), 1.51 (m, 6H), 2.01 (m, 1H), 2.28 (s, 3H), 2.39 (d, 2H), 7.51 (d, 1H), 8.41 (d, 1H), 8.52 (s, 1H), 12.41 (s, 1H).

### Beispiel 3

### 2-(Cyclohexylmethyl)-4-[(4-methyl-3-pyridinyl)amino]-6-oxo-1,6-dihydro-5-pyrimidincarbonitril

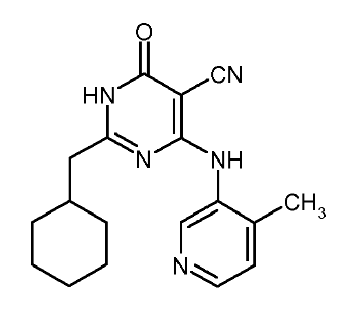

0.4 g (1.51 mmol) Methyl 2-cyano-3-[(4-methyl-3-pyridinyl)amino]-3-(methylsulfanyl)-2-propenoat werden mit 0.29 g (1.67 mmol) 2-Cyclohexylethanamidin-Hydrochlorid und 0.46 g (3.3 mmol) Kaliumcarbonat in 5 ml DMF gelöst und sieben Tage lang bei 90°C gerührt. Nach Abkühlen und Filtration wird das Produkt mittels präparativer HPLC gereinigt (YMC Gel ODS-AQ S 5/15 µm; Eluent A: Wasser, Eluent B: Acetonitril; Gradient: 0 min 30% B, 5 min 30% B, 50 min 95% B) gereinigt. Es werden 433 mg (88% d. Th.) des Produkts erhalten.
LC-MS (Methode 5): Rₜ = 1.47 min.
MS (ESIpos): m/z = 324 [M+H]⁺
¹H-NMR (300 MHz, DMSO-d₆): δ = 0.88 (m, 2H), 1.09 (m, 3H), 1.56 (m, 6H), 2.28 (d, 2H), 2.32 (s, 3H), 7.68 (s, 1H), 8.53 (d, 1H), 8.61 (s, 1H), 9.79 (s, 1H).

### Beispiel 4

### 2-(Cyclopentylmethyl)-4-[(3-fluorphenyl)amino]-6-oxo-1,6-dihydro-5-pyrimidincarbonitril

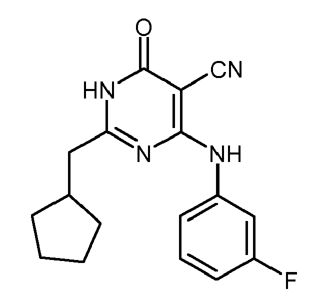

0.1 g (0.37 mmol) Methyl 2-cyano-3-[(3-fluorphenyl)amino]-3-(methylsulfanyl)-2-propenoat werden mit 0.06 g (0.41 mmol) 2-Cyclopentylethanamidin-Hydrochlorid in 1 ml DMF gelöst und mit 0.11 g (0.82 mmol) Kaliumcarbonat über Nacht auf 90°C erhitzt. Nach Filtration wird das Lösungsmittel im Vakuum entfernt und der Rückstand mittels präparativer HPLC gereinigt (YMC Gel ODS-AQ S 5/15 µm; Eluent A: Wasser, Eluent B: Acetonitril; Gradient: 0 min 30% B, 5 min 30% B, 50 min 95% B) gereinigt. Es werden 70 mg (59% d. Th.) des Produkts als farbloser Feststoff erhalten.
LC-MS (Methode 1): Rₜ = 2.9 min.
MS (ESIpos): m/z = 313 [M+H]⁺
¹H-NMR (300 MHz, DMSO-d₆): δ = 1.16 (m, 2H), 1.53 (m, 4H), 1.71 (m, 2H), 2.21 (m, 1H), 2.46 (d, 2H), 6.88 (m, 1H), 7.31 (m, 2H), 7.44 (m, 1H).

### Beispiel 5

### 4-[(3-Chlorphenyl)amino]-2-(cyclopentylmethyl)-6-oxo-1,6-dihydro-5-pyrimidincarbonitril

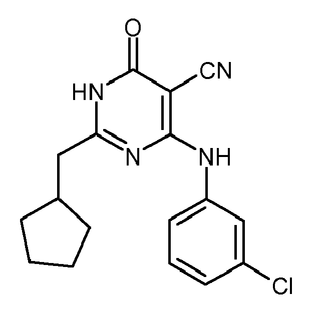

Analog zur Herstellung von Beispiel 4 werden aus 0.1 g (0.35 mmol) Methyl 2-cyano-3-[(3-chlorphenyl)amino]-3-(methylsulfanyl)-2-propenoat, 0.06 g (0.38 mmol) 2-Cyclopentylethanamidin-Hydrochlorid und 0.1 g (0.79 mmol) Kaliumcarbonat 45 mg (39% d. Th.) der Titelverbindung als farbloser Feststoff erhalten.
LC-MS (Methode 1): Rₜ = 3.06 min.
MS (ESIpos): m/z = 329 [M+H]⁺
¹H-NMR (300 MHz, DMSO-d₆): δ = 1.14 (m, 2H), 1.55 (m, 4H), 1.71 (m, 2H), 2.19 (m, 1H), 2.46 (d, 2H), 7.19 (m, 1H), 7.38 (m, 2H), 7.63 (m, 1H), 9.78 (br. s, 1H), 12.49 (br. s, 1H).

### Beispiel 6

### 2-(Cyclopentyhnethyl)-4-[(3-methoxyphenyl)amino]-6-oxo-1,6-dihydro-5-pyrimidincarbonitril

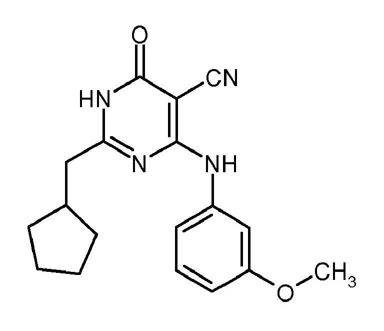

Analog zur Herstellung von Beispiel 4 werden aus 0.08 g (0.28 mmol) Methyl 2-cyano-3-[(3-methoxyphenyl)amino]-3-(methylsulfanyl)-2-propenoat, 0.05 g (0.31 mmol) 2-Cyclopentylethanamidin-Hydrochlorid und 0.09 g (0.63 mmol) Kaliumcarbonat 40 mg (42% d. Th.) der Titelverbindung als farbloser Feststoff erhalten.
LC-MS (Methode 1): Rₜ = 2.84 min.
MS (ESIpos): m/z = 325 [M+H]⁺.

### Beispiel 7

### 2-(Cyclopentyhnethyl)-4-[(3-fluor-2-methylphenyl)amino]-6-oxo-1,6-dihydro-5-pyrimidincarbonitril

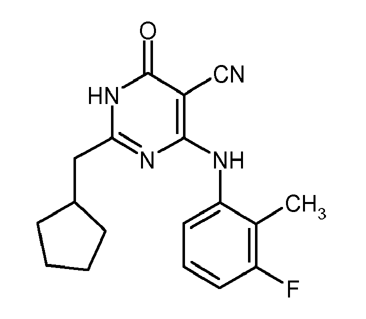

Analog zur Herstellung von Beispiel 4 werden aus 0.075 g (0.27 mmol) Methyl 2-cyano-3-[(3-fluor-2-methylphenyl)amino]-3-(methylsulfanyl)-2-propenoat, 0.047 g (0.29 mmol) 2-Cyclopentylethanamidin-Hydrochlorid und 0.081 g (0.59 mmol) Kaliumcarbonat 31 mg (35% d. Th.) der Titelverbindung als farbloser Feststoff erhalten.
LC-MS (Methode 6): Rₜ = 2.37 min.
MS (ESIpos): m/z = 327 [M+H]⁺
¹H-NMR (300 MHz, DMSO-d₆): δ = 1.08 (m, 2H), 1.43 (m, 4H), 1.55 (m, 2H), 2.04 (m, 1H), 2.04 (s, 3H), 2.39 (d, 2H), 7.05 (m, 2H), 7.20 (m, 1H), 9.60 (s, 1H), 12.30 (s, 1H).

### Beispiel 8

### 2-(Cyclopentylmethyl)-4-[(2,5-dimethylphenyl)amino]-6-oxo-1,6-dihydro-5-pyrimidincarbonitril

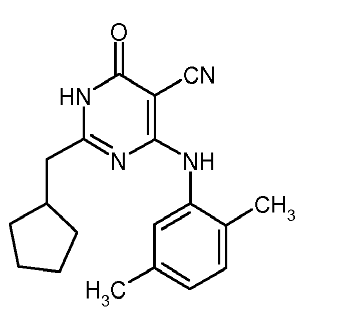

Analog zur Herstellung von Beispiel 4 werden aus 0.1 g (0.37 mmol) Methyl 2-cyano-3-[(2,5-dimethylphenyl)amino]-3-(methylsulfanyl)-2-propenoat, 0.06 g (0.39 mmol) 2-Cyclopentylethanamidin-Hydrochlorid und 0.1 g (0.79 mmol) Kaliumcarbonat 53 mg (45% d. Th.) der Titelverbindung als farbloser Feststoff erhalten.
LC-MS (Methode 10): Rₜ = 3.39 min.
MS (ESIpos): m/z = 323 [M+H]⁺
¹H-NMR (300 MHz, DMSO-d₆): δ = 1.10 (m, 2H), 1.43 (m, 4H), 1.62 (m, 2H), 2.04 (m, 1H), 2.10 (s, 3H), 2.24 (s, 3H), 2.36 (d, 2H), 6.98 (m, 1H), 7.09 (m, 2H), 9.15 (br. s, 1H), 12.19 (br. s, 1H).

### Beispiel 9

### 2-(Cyclopentylmethyl)-4-[(2-methoxyphenyl)amino]-6-oxo-1,6-dihydro-5-pyrimidincarbonitril

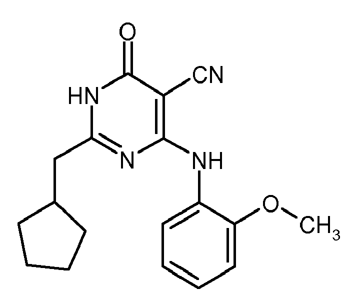

Analog zur Herstellung von Beispiel 4 werden aus 0.2 g (0.71 mmol) Methyl 2-cyano-3-[(2-methoxyphenyl)amino]-3-(methylsulfanyl)-2-propenoat, 0.13 g (0.79 mmol) 2-Cyclopentylethanamidin-Hydrochlorid und 0.22 g (1.58 mmol) Kaliumcarbonat 62 mg (26% d. Th.) der Titelverbindung als farbloser Feststoff erhalten.
LC-MS (Methode 1): Rₜ = 2.92 min.
MS (ESIpos): m/z = 325 [M+H]⁺.

### Beispiel 10

### 2-(Cyclopentylmethyl)-4-[(4-fluor-2-methylphenyl)amino]-6-oxo-1,6-dihydro-5-pyrimidincarbonitril

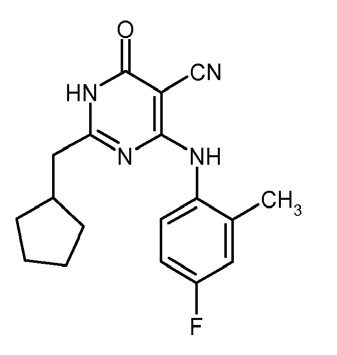

Analog zur Herstellung von Beispiel 4 werden aus 0.1 g (0.36 mmol) Methyl 2-cyano-3-[(4-fluor-2-methylphenyl)amino]-3-(methylsulfanyl)-2-propenoat, 0.06 g (0.39 mmol) 2-Cyclopentylethanamidin-Hydrochlorid und 0.1 g (0.78 mmol) Kaliumcarbonat 54 mg (46% d. Th.) der Titelverbindung als farbloser Feststoff erhalten.
LC-MS (Methode 3): Rₜ = 2.98 min.
MS (ESIpos): m/z = 327 [M+H]⁺
¹H-NMR (300 MHz, DMSO-d₆): δ = 1.09 (m, 2H), 1.53 (m, 6H), 2.10 (m, 1H), 2.14 (s, 3H), 2.28 (d, 2H), 6.98 (m, 1H), 7.08 (m, 1H), 7.29 (m, 1H).

### Beispiel 11

### 2-(Cyclopentylmethyl)-4-[(2-methylphenyl)amino]-6-oxo-1,6-dihydro-5-pyrimidincarbonitril

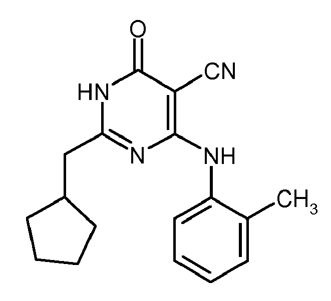

Analog zur Herstellung von Beispiel 4 werden aus 2.48 g (9.45 mmol) Methyl 2-cyano-3-[(2-methylphenyl)amino]-3-(methylsulfanyl)-2-propenoat, 1.69 g (10.33 mmol) 2-Cyclopentylethanamidin-Hydrochlorid und 2.87 g (20.79 mmol) Kaliumcarbonat 1.25 g (43% d. Th.) der Titelverbindung als farbloser Feststoff erhalten.
LC-MS (Methode 1): Rₜ = 2.84 min.
MS (ESIpos): m/z = 309 [M+H]⁺
¹H-NMR (300 MHz, DMSO-d₆): δ = 1.07 (m, 2H), 1.42 (m, 2H), 1.52 (m, 2H), 1.59 (m, 2H), 2.05 (m, 1H), 2.14 (s, 3H), 2.36 (d, 2H), 7.17 (m, 4H), 9.47 (s, 1H), 12.24 (s, 1H).

### Beispiel 12

### 2-(Cyclopentylmethyl)-6-oxo-4-[(2-propylphenyl)amino]-1,6-dihydro-5-pyrimidincarbonitril

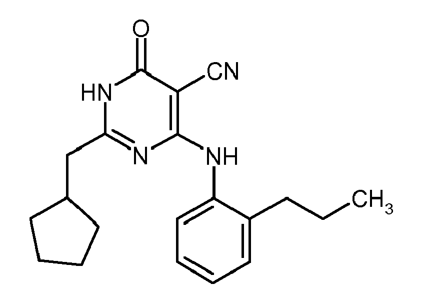

Analog zur Herstellung von Beispiel 4 werden aus 0.1 g (0.34 mmol) Methyl 2-cyano-3-(methyl-sulfanyl)-3-[(2-propylphenyl)amino]-2-propenoat, 0.06 g (0.37 mmol) 2-Cyclopentylethanamidin-Hydrochlorid und 0.1 g (0.76 mmol) Kaliumcarbonat 57 mg (49% d. Th.) der Titelverbindung als farbloser Feststoff erhalten.
LC-MS (Methode 1): Rₜ = 3.13 min.
MS (ESIpos): m/z = 337 [M+H]⁺
¹H-NMR (300 MHz, DMSO-d₆): δ = 0.84 (t, 3H), 1.07 (m, 2H), 1.52 (m, 8H), 2.04 (m, 1H), 2.35 (d, 2H), 2.49 (m, 2H), 7.16 (m, 2H), 7.23 (m, 2H), 9.44 (s, 1H), 12.20 (s, 1H).

### Beispiel 13

### 2-(Cyclopentyhnethyl)-6-oxo-4-(3-pyridinylamino)-1,6-dihydro-5-pyrimidincarbonitril

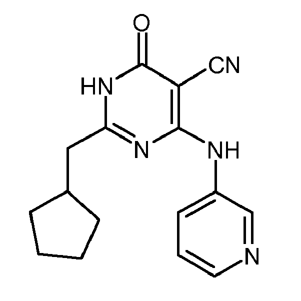

0.1 g (0.40 mmol) Methyl 2-cyano-3-(methylsulfanyl)-3-(3-pyridinylamino)-2-propenoat werden mit 0.065 g (0.40 mmol) 2-Cyclopentylethanamidin-Hydrochlorid und 0.16 g (1.60 mmol) Triethylamin in 0.5 ml DMF gelöst und über Nacht bei 100°C gerührt. Nach dem Abkühlen wird der Ansatz mit 10 ml Wasser verdünnt und mit Dichlormethan extrahiert. Die organische Phase wird über Natriumsulfat getrocknet, im Vakuum eingeengt und der Rückstand über Flash-Chromatographie (Laufmittel: Dichlormethan/Methanol 200:1, 100:1, 50:1) gereinigt. Es werden 78 mg (64% d. Th.) des Produkts als farbloser Feststoff erhalten.
HPLC (Methode 8): Rₜ = 3.36 min.
MS (ESIpos): m/z = 296 [M+H]⁺
¹H-NMR (300 MHz, DMSO-d₆): δ = 1.05-1.20 (m, 2H), 1.37-1.75 (m, 6H), 2.16 (m, 1H), 2.47 (d, 2H), 7.36 (m, 1H), 7.83 (m, 1H), 8.32 (m, 1H), 8.66 (m, 1H), 9.80 (s, 1H), 12.48 (s, 1H).

### Beispiel 14

### 2-(2-Methylbutyl)-4-[(2-methylphenyl)amino]-6-oxo-1,6-dihydro-5-pyrimidincarbonitril

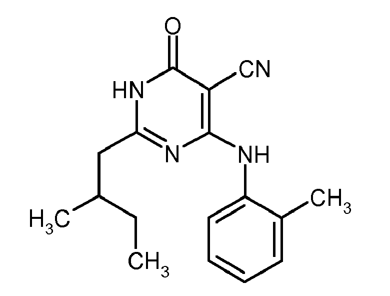

Analog zur Herstellung von Beispiel 4 werden aus 0.12 g (0.46 mmol) Methyl 2-cyano-3-(methyl-sulfanyl)-3-[(2-methylphenyl)amino]-2-propenoat, 0.07 g (0.50 mmol) 3-Methylpentanamidin-Hydrochlorid und 0.14 g (1.0 mmol) Kaliumcarbonat 105 mg (77% d. Th.) der Titelverbindung als farbloser Feststoff erhalten.
LC-MS (Methode 2): Rₜ = 2.06 min.
MS (ESIpos): m/z = 297 [M+H]⁺
¹H-NMR (300 MHz, DMSO-d₆): δ = 0.80 (d, 6H), 1.09 (m, 1H), 1.24 (m, 1H), 1.74 (m, 1H), 2.14 (s, 3H), 2.32 (dd, 2H), 7.16 (m, 4H), 9.49 (s, 1H), 12.27 (s, 1H).

### Beispiel 15

### 2-[(2S)-2-Methylbutyl]-4-[(2-methylphenyl)amino]-6-oxo-1,6-dihydro-5-pyrimidincarbonitril

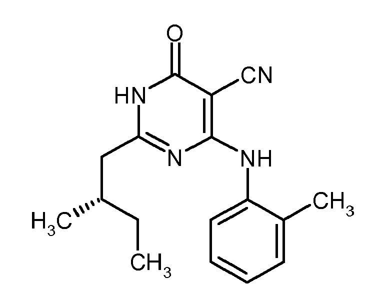

Analog zur Herstellung von Beispiel 4 werden aus 0.2 g (0.76 mmol) Methyl 2-cyano-3-(methyl-sulfanyl)-3-[(2-methylphenyl)amino]-2-propenoat, 0.17 g (1.14 mmol) (3*S*)-3-Methylpentanamidin-Hydrochlorid und 0.23 g (1.66 mmol) Kaliumcarbonat 183 mg (80% d. Th.) der Titelverbindung als farbloser Feststoff erhalten.
LC-MS (Methode 5): Rₜ = 2.28 min.
MS (ESIpos): m/z = 297 [M+H]⁺
¹H-NMR (200 MHz, DMSO-d₆): δ = 0.78 (d, 6H), 1.09 (m, 1H), 1.21 (m, 1H), 1.70 (m, 1H), 2.13 (s, 3H), 2.29 (dd, 2H), 7.16 (m, 4H), 9.49 (s, 1H), 12.25 (s, 1H).

### Beispiel 16

### 4-[(5-Fluor-2-methylphenyl)amino]-2-[(2S)-2-methylbutyl]-6-oxo-1,6-dihydro-5-pyrimidincarbonitril

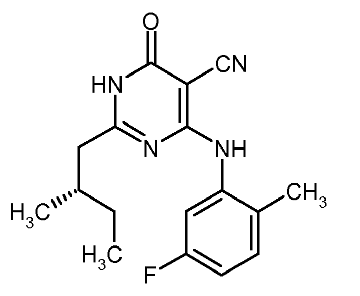

Analog zur Herstellung von Beispiel 4 werden aus 0.22 g (0.78 mmol) Methyl 2-cyano-3-[(5-fluor-2-methylphenyl)amino]-3-(methylsulfanyl)-2-propenoat, 0.17 g (1.17 mmol) (3*S*)-3-Methylpentan-amidin-Hydrochlorid und 0.24 g (1.73 mmol) Kaliumcarbonat 182 mg (73% d. Th.) der Titelverbindung als farbloser Feststoff erhalten.
LC-MS (Methode 5): Rₜ = 2.11 min.
MS (ESIpos): m/z = 315 [M+H]⁺
¹H-NMR (200 MHz, DMSO-d₆): δ = 0.75 (d, 6H), 1.1 (m, 1H), 1.25 (m, 1H), 1.71 (m, 1H), 2.1 (s, 3H), 2.26 (dd, 2H), 7.03 (m, 2H), 7.28 (m, 1H), 9.51 (s, 1H), 12.36 (s, 1H).

Analog zur Herstellung von Beispiel 4 werden die in der folgenden Tabelle aufgeführten Beispiele 17-58 dargestellt:

| **Beispiel** | **Struktur** | **MS: m/z [M+H]⁺** | **Rₜ [min]** | **HPLC**- **bzw**. **LC-MS-Methode** |
|---|---|---|---|---|
| **17** | 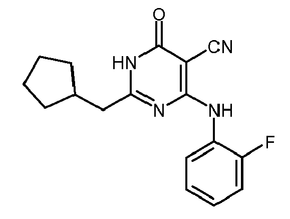 | 313 | 4.37 | 8 |
| **18** | 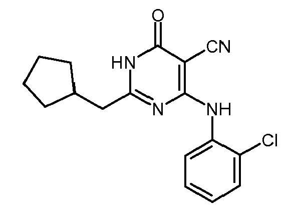 | 329 | 4.52 | 8 |
| **19** | 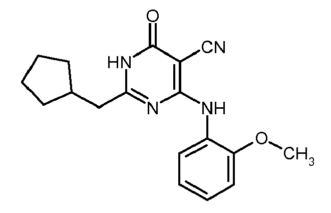 | 325 | 4.50 | 8 |
| **20** | 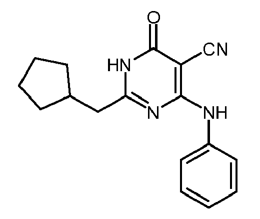 | 295 | 4.39 | 8 |
| **21** | 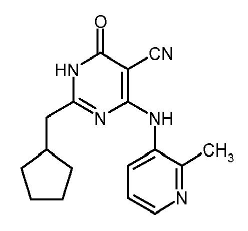 | 310 | 1.82 | 1 |
| **22** | 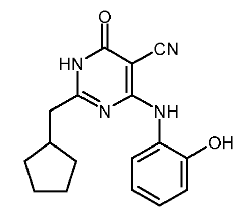 | 311 | 2.75 | 7 |
| **23** | 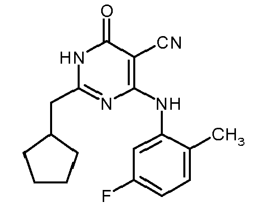 | 327 | 2.39 | 6 |
| **24** | 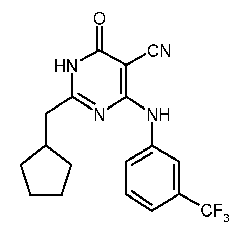 | 363 | 3.12 | 1 |
| **25** | 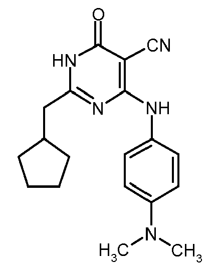 | 338 | 2.87 | 10 |
| **26** | 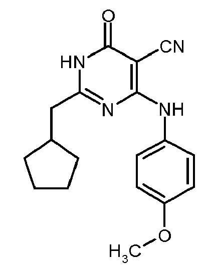 | 325 | 2.78 | 1 |
| **27** | 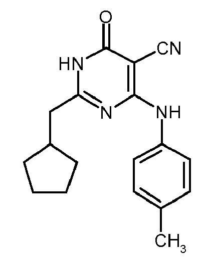 | 309 | 2.97 | 1 |
| **28** | 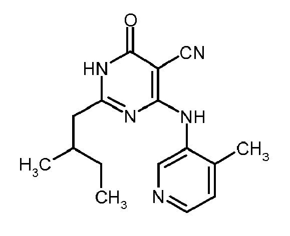 | 298 | 1.26 | 2 |
| **29** | 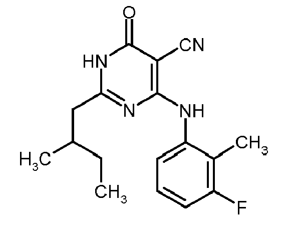 | 315 | 2.26 | 6 |
| **30** | 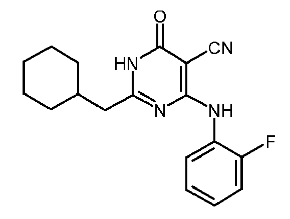 | 327 | 4.52 | 8 |
| **31** | 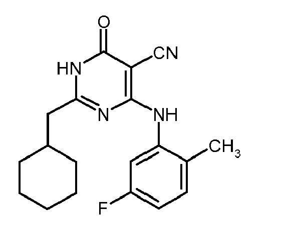 | 341 | 2.51 | 6 |
| **32** | 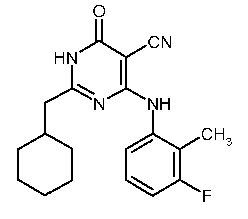 | 341 | 2.48 | 6 |
| **33** | 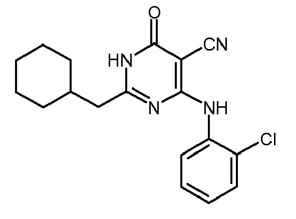 | 343 | 4.67 | 8 |
| **34** | 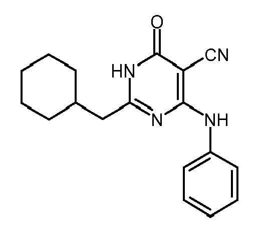 | 309 | 4.55 | 8 |
| **35** | 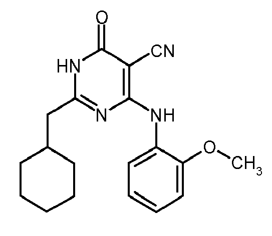 | 339 | 4.67 | 8 |
| **36** | 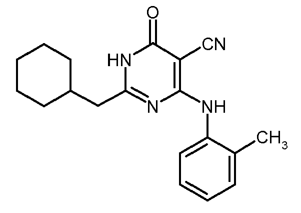 | 340 ([M+NH₄]⁺) | 4.61 | 8 |
| **37** | 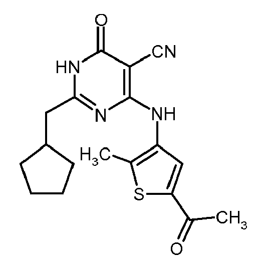 | 357 | 2.7 | 1 |
| **38** | 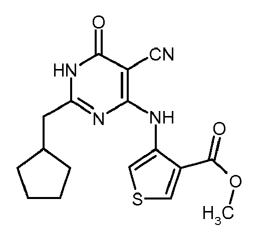 | 359 | 3.3 | 7 |
| **39** | 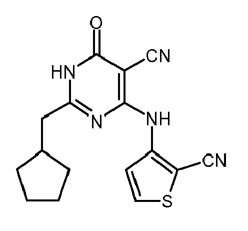 | 326 | 2.23 | 9 |
| **40** | 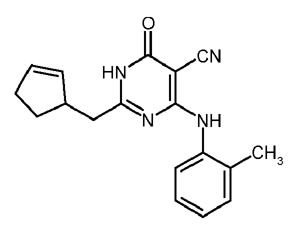 | 307 | 4.35 | 8 |
| **41** | 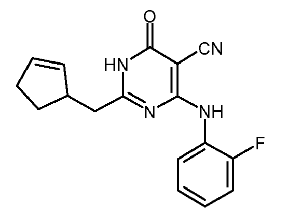 | 311 | 4.25 | 8 |
| **42** | 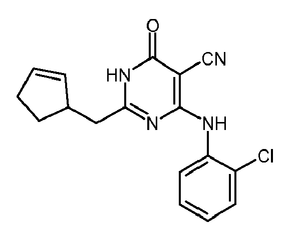 | 327 | 4.40 | 8 |
| **43** | 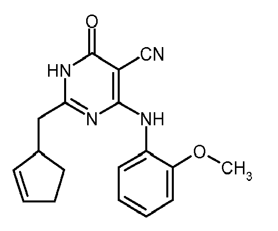 | 323 | 4.39 | 8 |
| **44** | 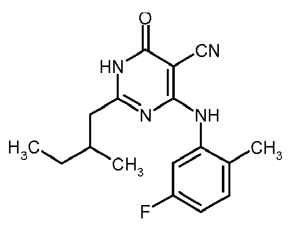 | 315 | 2.34 | 6 |
| **45** | 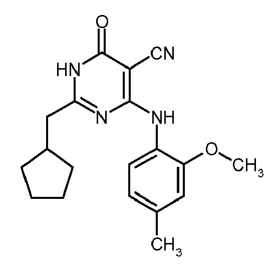 | 339 | 2.3 | 2 |
| **46** | 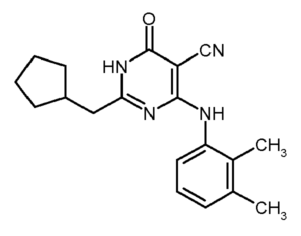 | 323 | 2.18 | 2 |
| **47** | 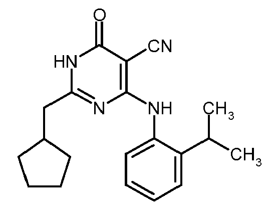 | 337 | 2.31 | 2 |
| **48** | 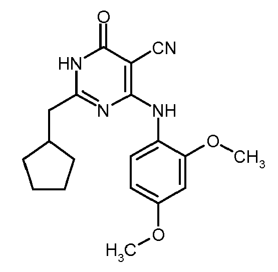 | 355 | 2.09 | 2 |
| **49** | 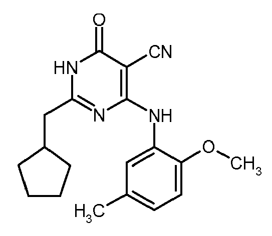 | 339 | 2.33 | 2 |
| **50** | 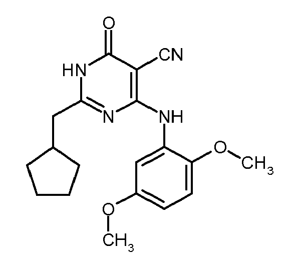 | 355 | 2.20 | 2 |
| **51** | 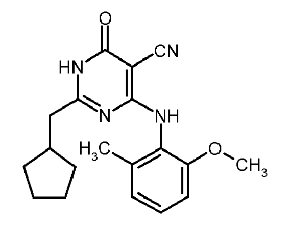 | 339 | 2.05 | 2 |
| **52** | 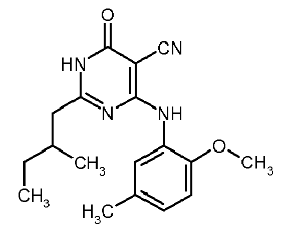 | 327 | 2.48 | 5 |
| **53** | 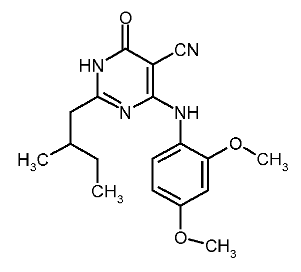 | 343 | 2.25 | 5 |
| **54** | 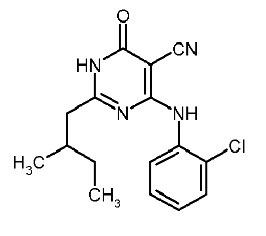 | 317 | 2.32 | 5 |
| **55** | 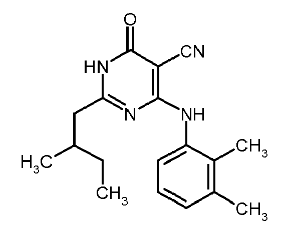 | 311 | 2.34 | 5 |
| **56** | 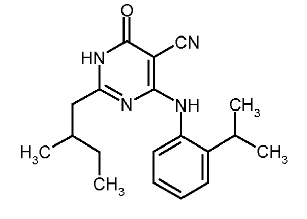 | 325 | 2.26 | 2 |
| **57** | 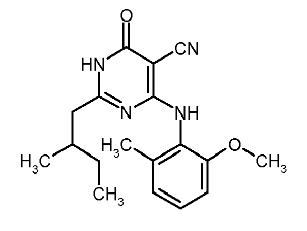 | 327 | 2.23 | 5 |
| **58** | 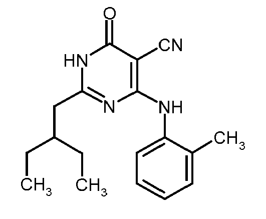 | 311 | 2.40 | 5 |
| **59** | 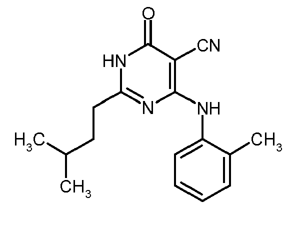 | 297 | 4.40 | 8 |

## Patentansprüche

1. Verbindungen der Formel 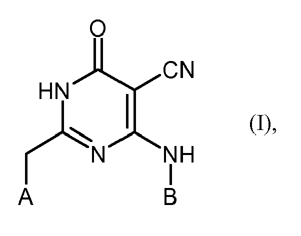 in welcher
A C₁-C₈-Alkyl, C₃-C₈-Cycloalkyl, Tetrahydrofuryl oder Tetrahydropyranyl, welche gegebenenfalls mit bis zu 3 Resten unabhängig voneinander ausgewählt aus der Gruppe C₁-C₆-Alkyl, C₁-C₆-Alkoxy, Hydroxycarbonyl, Cyano, Trifluormethyl, Trifluormethoxy, Amino, Hydroxy, C₁-C₆-Alkylamino, Halogen, C₁-C₆-Alkylaminocarbonyl, C₁-C₆-Alkoxycarbonyl, C₁-C₆-Alkylcarbonyl, C₁-C₆-Alkylsulfonyl und C₁-C₆-Alkylthio substituiert sind,
wobei C₁-C₆-Alkyl, C₁-C₆-Alkoxy, C₁-C₆-Alkylamino, C₁-C₆-Alkylaminocarbonyl, C₁-C₆-Alkoxycarbonyl, C₁-C₆-Alkylcarbonyl, C₁-C₆-Alkylsulfonyl und C₁-C₆-Alkylthio gegebenenfalls mit einem oder mehreren Resten ausgewählt aus der Gruppe Hydroxy, Cyano, Halogen, Hydroxycarbonyl und einer Gruppe der Formel -NR³R⁴,
wobei
R³ und R⁴ unabhängig voneinander Wasserstoff oder C₁-C₆-Alkyl,
oder
R³ und R⁴ zusammen mit dem Stickstoffatom, an das sie gebunden sind, 5-bis 8-gliedriges Heterocyclyl bedeuten,
substituiert sind,
B Phenyl oder Heteroaryl, die gegebenenfalls mit bis zu 3 Resten jeweils unabhängig voneinander ausgewählt aus der Gruppe C₁-C₆-Allcyl, C₁-C₆-Alkoxy, Hydroxycarbonyl, Cyano, Trifluormethyl, Trifluormethoxy, Amino, Nitro, Hydroxy, C₁-C₆-Alkylamino, Halogen, C₁-C₆-Alkylaminocarbonyl, C₁-C₆-Alkoxycarbonyl, C₁-C₆-Alkylcarbonyl, C₁-C₆-Alkylsulfonyl und C₁-C₆-Alkylthio substituiert sind,
wobei C₁-C₆-Alkyl, C₁-C₆-Alkoxy, C₁-C₆-Alkylamino, C₁-C₆-Alkylaminocarbonyl, C₁-C₆-Alkoxycarbonyl, C₁-C₆-Alkylcarbonyl, C₁-C₆-Alkylsulfonyl und C₁-C₆-Alkylthio gegebenenfalls mit einem Rest ausgewählt aus der Gruppe Hydroxy, Cyano, Halogen, Hydroxycarbonyl und einer Gruppe der Formel
- NR³R⁴,
wobei
R³ und R⁴ die oben angegebenen Bedeutungen aufweisen,
substituiert sind,
bedeuten, sowie deren Salze, Solvate und/oder Solvate der Salze.

2. Verbindungen nach Anspruch 1, wobei
A C₁-C₅-Alkyl oder C₃-C₆-Cycloalkyl, welche gegebenenfalls mit bis zu 3 Resten unabhängig voneinander ausgewählt aus der Gruppe C₁-C₄-Alkyl, C₁-C₄-Alkoxy, Hydroxycarbonyl, Cyano, Amino, Hydroxy, C₁-C₄-Alkylamino, Fluor, Chlor, Brom, C₁-C₄-Alkoxycarbonyl, C₁-C₆-Alkylcarbonyl, C₁-C₄-Allcylsulfonyl und C₁-C₄-Alkylthio substituiert sind,
wobei C₁-C₄-Alkyl und C₁-C₄-Alkoxy gegebenenfalls mit einem Rest ausgewählt aus der Gruppe Hydroxy, Cyano, Fluor, Chlor, Brom, Hydroxycarbonyl und einer Gruppe der Formel -NR³_{R}⁴,
wobei
R³ und R⁴ unabhängig voneinander Wasserstoff oder C₁-C₄-Alkyl,
oder
R³ und R⁴ zusammen mit dem Stickstoffatom, an das sie gebunden sind, 5-bis 6-gliedriges Heterocyclyl bedeuten,
substituiert sind,
B Phenyl, Thienyl oder Pyridyl, die gegebenenfalls mit bis zu 3 Resten jeweils unabhängig voneinander ausgewählt aus der Gruppe C₁-C₄-Alkyl, C₁-C₄-Alkoxy, Hydroxycarbonyl, Cyano, Trifluormethyl, Trifluormethoxy, Amino, Hydroxy, C₁-C₄-Alkylamino, Fluor, Chlor, Brom, C₁-C₄-Alkylaminocarbonyl, C₁-C₄-Alkoxycarbonyl, C₁-C₄-Alkylcarbonyl, C₁-C₄-Alkylsulfonyl und C₁-C₄-Alkylthio substituiert sind,
wobei C₁-C₄-Alkyl und C₁-C₄-Alkoxy gegebenenfalls mit einem Rest ausgewählt aus der Gruppe Hydroxy, Cyano, Fluor, Chlor, Brom, Hydroxycarbonyl und einer Gruppe der Formel -NR³R⁴,
wobei
R³ und R⁴ die oben angegebenen Bedeutungen aufweisen,
substituiert sind,
bedeuten, sowie deren Salze, Solvate und/oder Solvate der Salze.

3. Verbindungen nach Ansprüchen 1 und 2, wobei
A C₃-C₅-Alkyl oder C₅-C₆-Cycloalkyl,
B Phenyl, Thienyl oder Pyridyl, die gegebenenfalls mit bis zu 3 Resten jeweils unabhängig voneinander ausgewählt aus der Gruppe C₁-C₃-Alkyl, Trifluormethyl, Hydroxy, Methoxy, Ethoxy, Cyano, Dimethylamino, Diethylamino, Methoxycarbonyl, Ethoxycarbonyl, Methylcarbonyl, Ethylcarbonyl, Fluor und Chlor substituiert sind,
bedeuten, sowie deren Salze, Solvate und/oder Solvate der Salze.

4. Verfahren zur Herstellung von Verbindungen der Formel (I), **dadurch gekennzeichnet, dass** man Verbindungen der Formel 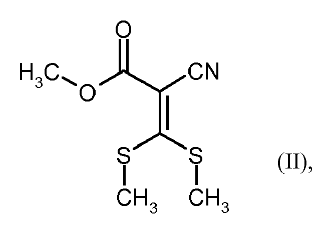 zunächst mit einer Verbindung der Formel
H₂N-B (III)
,
in welcher
B die in Ansprüchen 1 bis 3 angegebenen Bedeutungen aufweist,
bei erhöhter Temperatur in einem inerten Lösemittel oder auch in Abwesenheit eines Lösemittels in eine Verbindung der Formel 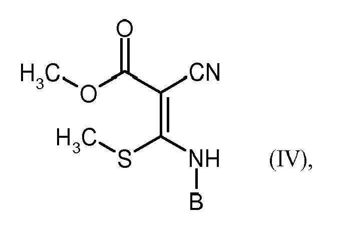 in welcher
B die in Ansprüchen 1 bis 3 angegebenen Bedeutungen aufweist,
überführt und diese dann in einem inerten Lösemittel in Gegenwart einer Base mit einer Verbindung der Formel 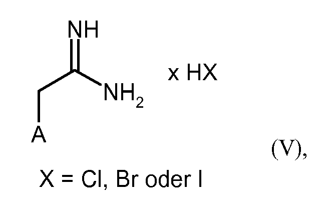 in welcher
A die in Ansprüchen 1 bis 3 angegebenen Bedeutungen aufweist,
umsetzt,
und die resultierenden Verbindungen der Formel (I) gegebenenfalls mit den entsprechenden (i) Lösemitteln und/oder (ii) Basen oder Säuren zu ihren Solvaten, Salzen und/oder Solvaten der Salze umsetzt.

5. Verbindungen nach einem der Ansprüche 1 bis 3 zur Behandlung und/oder Prophylaxe von Krankheiten.

6. Arzneimittel enthaltend mindestens eine der Verbindungen nach einem der Ansprüche 1 bis 3 und mindestens einen pharmazeutisch verträglichen, im wesentlichen nichtgiftigen Träger oder Exzipienten.

7. Verwendung der Verbindungen nach einem der Ansprüche 1 bis 3 zur Herstellung eines Arzneimittels zur Prophylaxe und/oder Behandlung von Störungen der Wahrnehmung, Konzentrationsleistung, Lern- und/oder Gedächtnisleistung.

8. Verwendung nach Anspruch 7, wobei die Störung eine Folge der Alzheimer'schen Krankheit ist.

9. Verwendung der Verbindungen nach einem der Ansprüche 1 bis 3 zur Herstellung eines Arzneimittels zur Verbesserung der Wahrnehmung, Konzentrationsleistung, Lern- und/oder Gedächtnisleistung.

10. Verfahren zur Bekämpfung von Störungen der Wahrnehmung, Konzentrationsleistung, Lern-und/oder Gedächtnisleistung in Mensch oder Tier durch Verabreichung einer wirksamen Menge der Verbindungen aus Ansprüchen 1 bis 3.

11. Verfahren nach Anspruch 10, wobei die Störung eine Folge der Alzheimer'schen Krankheit ist.
